(19) Europäisches Patentamt / European Patent Office / Office européen des brevets

(11) **EP 3 419 511 B1**

(12) **EUROPEAN PATENT SPECIFICATION**

(45) Date of publication and mention
of the grant of the patent:
**23.10.2024 Bulletin 2024/43**

(21) Application number: **17757437.3**

(22) Date of filing: **27.02.2017**

(51) International Patent Classification (IPC):
***A61B 5/02*** *(2006.01)*       ***A61B 5/0285*** *(2006.01)*
***A61B 5/021*** *(2006.01)*

(52) Cooperative Patent Classification (CPC):
**A61B 5/02125; A61B 5/0285; A61B 5/681;**
**A61B 5/7257;** A61B 5/7203; A61B 2505/07;
A61B 2562/043

(86) International application number:
**PCT/US2017/019775**

(87) International publication number:
**WO 2017/147609 (31.08.2017 Gazette 2017/35)**

(54) **SYSTEMS AND METHODS FOR MODIFIED PULSE TRANSIT TIME MEASUREMENT**

SYSTEME UND VERFAHREN ZUR MODIFIZIERTEN IMPULSLAUFZEITMESSUNG

SYSTÈMES ET PROCÉDÉS DE MESURE DE LA DURÉE DE TRANSIT D'IMPULSION MODIFIÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.02.2016 US 201662299925 P**

(43) Date of publication of application:
**02.01.2019 Bulletin 2019/01**

(73) Proprietor: **Itamar Medical Spry 2021, Limited
Partnership
3088900 Caesarea (IL)**

(72) Inventors:
• **FERBER, Elad
Woodside
California 94062 (US)**
• **NARAYANAN, Ramkrishnan
San Jose
California 95118 (US)**
• **GUNGOR, Derya Gol
Sunnyvale
California 94085 (US)**

(74) Representative: **Potter Clarkson
Chapel Quarter
Mount Street
Nottingham NG1 6HQ (GB)**

(56) References cited:
| | |
|---|---|
| EP-A1- 2 631 874 | WO-A2-2009/125349 |
| US-A1- 2002 058 876 | US-A1- 2002 058 876 |
| US-A1- 2008 294 058 | US-A1- 2008 294 058 |
| US-A1- 2013 137 938 | US-A1- 2013 137 938 |
| US-A1- 2014 249 442 | US-A1- 2016 242 700 |

**Description**

**BACKGROUND**

Technical Field

Embodiments relate generally to blood metrics measurement. More specifically, embodiments relate to modified pulse transit time measurement and non-invasive blood pressure measurement.

Description of Related Art

Wearable activity monitoring devices are growing in popularity. These devices aim to facilitate achieving a user's goal such as to lose weight, to increase physical activity, or simply to improve overall health. Many such devices may interface with computer software to allow visualization of the recorded data. Nevertheless, most devices are evolved cousins of pedometers, which measure the number of steps a user takes. Even though additional functions such as tallying the distance a user travels or calculating calorie consumptions may be added, these devices lack the ability to measure blood metrics.

Blood pressure is an important factor in both heart health and overall health. For example, elevated blood pressure may result in coronary artery disease, heart failure and hypertrophy. Accordingly, blood pressure monitoring has become an important component of patient health. Typically, blood pressure is monitored using a blood pressure gauge with an inflatable cuff. However, such devices are often uncomfortable and unable to provide continuous blood pressure measurement.

US 2008/0294058 A1 discloses a wearable device for measuring a pulse while a user is in motion. US 2013/0137938 A1 discloses a patient monitor which apply phase detection operations to analog signals to identify differential pulse transit time (DPTT), which may then be used to determine, for example, the subject's blood pressure.

SUMMARY

According to aspects of the disclosure we provide a wearable blood metrics measurement apparatus, a method and a non-transitory computer readable medium as defined in the independent claims. We also disclose various unclaimed aspects useful for understanding. Various embodiments of the present disclosure include systems, methods, and non-transitory computer readable media configured to select at least two signal channels corresponding to an arterial signal, the at least two signal channels associated with corresponding optical sensors of the wearable blood metrics measurement apparatus. First signal data is obtained from a first channel of the at least two signal channels over a predetermined period of time. Second signal data is obtained from a second channel of the at least two signal channels over the predetermined period of time. A frequency transform function (e.g., Fourier transform) is applied to each of the first signal data and the second signal data to transform the first signal data and the second signal data to a frequency domain. A first phase value is determined for a first frequency component of the first signal data in the frequency domain. A second phase value is determined for a second frequency component of the second signal data in the frequency domain. A phase difference value is determined between the first phase value and the second phase value. A time shift value is determined between the first signal data and the second signal data based on the phase difference value. A modified pulse transmit time is determined based on the time shift value between the first signal data and the second signal data, the modified pulse transit time representing a transit time for a pressure wavefront to travel from a first optical sensor of the corresponding optical sensors and a second optical sensor of the corresponding optical sensors. A pulse wave velocity is determined based on the modified pulse transit time. An arterial blood pressure value is calculated based on the pulse wave velocity; and a message is provided including or being based on the arterial blood pressure value.

The corresponding optical sensors of the wearable blood metrics measurement apparatus are selected from one or more arrays of optical sensors. In related embodiments, the selecting at least two signal channels corresponding to the arterial signal includes obtaining time series data for each of a plurality of channel pairs associated with the one or more array of optical sensors; calculating a correlation coefficient between each of the time series data; determining a correlation value for each channel pair, each correlation value of a respective channel pair representing a correlation between a first and second channel of the respective channel pair; and selecting the channel pair having the highest correlation value relative to the correlation values of the other channel pairs of the plurality of channel pairs, the selected channel pair comprising the at least two signal channels corresponding to the at least two signal channels corresponding to the arterial signal.

In some embodiments, the selecting at least two signal channels corresponding to the arterial signal further includes applying a high pass filter to remove selected motion artifacts prior to calculating the correlation coefficient.

In some embodiments, the one or more arrays of optical sensors comprise two rows of optical sensor pairs separated by a predetermined distance, and the pulse wave velocity is determined based on dividing the predetermined distance by the modified pulse transit time.

In some embodiments, the obtaining the first signal data includes projecting, by an energy transmitter of the wearable blood metrics measurement apparatus, energy at a first wavelength into tissue of a user; and generating the first signal data based on a first received portion of the energy at the first wavelength, the first received portion of energy being received through the tissue of the user. In related embodiments, the obtaining the second signal data includes projecting, by the energy transmitter of the wearable blood metrics measurement apparatus, energy at a second wavelength into tissue of the user; generating the second signal data based on a second received portion of the energy at the second wavelength, the second received portion of energy being received through the tissue of the user. In some embodiments, the first wavelength and the second wavelength comprise the same wavelength.

In some embodiments, the first signal data and the second signal data each comprise photoplethysmogram (PPG) signal data.

In various embodiments, an example system comprises an energy transmitter, an energy receiver, and an analyzer. The energy transmitter may project energy at a first wavelength and a second wavelength into tissue of a user, the first wavelength and the second wavelength being associated with at least one nutrient of a set of nutrients in blood of the user. The energy receiver may generate a composite signal based on a fraction of the energy at the first wavelength and the second wavelength, the fraction of the energy being received through the tissue of the user. The analyzer may separate the composite signal into a first signal corresponding to the first wavelength and a second signal corresponding to the second wavelength, and detect, in the blood of the user, a concentration of the at least one nutrient of the set of nutrients based on the first signal and the second signal.

The fraction of the energy may be received by the energy receiver after the fraction of the energy is reflected by the tissue of the user. The system may comprise a wearable member. The energy transmitter and the energy receiver may be secured to the wearable member such that the energy transmitter and the energy receiver are in contact or in proximity with the tissue. The analyzer may be further configured to determine a set of blood metrics based on the first signal and the second signal, the concentration of at least one nutrient of the set of nutrients being determined based on the determined set of blood metrics. The system may further comprise a user interface configured to display at least some of the set of blood metrics. The analyzer may be further configured to compare a blood metric of the set of blood metric to a threshold and to generate an alert if the blood metric exceeds the threshold. The set of blood metrics may comprise a blood glucose concentration.

The analyzer may be further configured to determine a first AC component and a first DC component of the first signal, to determine a second AC component and a second DC component of the second signal, wherein the concentration of a nutrient of the set of nutrients is detected based on the first AC component, the first DC component, the second AC component, and the second DC component. The system may further comprise a motion detector configured to measure a level of motion, and the analyzer is configured to compare the level of motion to a threshold and to discount a measurement of the composite signal when the level of motion exceeds the threshold. A nutrient of the set of nutrients may comprise glucose.

An example method may comprise projecting energy at a first wavelength and a second wavelength into tissue of a user, the first wavelength and the second wavelength being associated with at least one nutrient of a set of nutrients in blood of the user, generating a composite signal based on a fraction of the energy at the first wavelength and the second wavelength, the fraction of the energy being received through the tissue of the user, separating the composite signal into a first signal corresponding to the first wavelength and a second signal corresponding to the second wavelength, and detecting, in the blood of the user, a concentration of the at least one nutrient of the set of nutrients based on the first signal and the second signal.

Another example system may comprise an energy transmitter, an energy receiver, and an analyzer. The energy transmitter may be configured to project energy at a first wavelength and a second wavelength into tissue of a user, the first wavelength and the second wavelength being associated with, in blood of the user, at least one component. The at least one component being at least one of one of glucose, hemoglobin, triglycerides, cholesterol, bilirubin, protein, albumin, blood pH, Hematocrit, cortisol, and/or electrolytes. The energy receiver may be configured to generate a composite signal based on a fraction of the energy at the first wavelength and the second wavelength, the fraction of the energy being received through the tissue of the user. The analyzer may be configured to separate the composite signal into a first signal corresponding to the first wavelength and a second signal corresponding to the second wavelength, and to detect, in the blood of the user, a concentration of the at least one component based on the first signal and the second signal.

Other features and aspects of various embodiments will become apparent from the following detailed description, taken in conjunction with the accompanying drawings, which illustrate, by way of example, the features of such embodiments.

BRIEF DESCRIPTION OF THE DRAWINGS

FIG. 1 depicts a graph showing an example pulse transit time according to some embodiments.

FIG. 2 depicts an example blood metrics measurement apparatus according to some embodiments.

FIG. 3A depicts a block diagram of an example blood metrics measurement apparatus according to some embodiments.

FIGS. 3B-C depict block diagram of example sensor systems according to some embodiments.

FIG. 4 depicts a graph of an example waves from each LED-photodiode pair for six signal channels.

FIG. 5 depicts a flowchart of an example method of operation of a blood metrics measurement apparatus according to some embodiments.

FIG. 6 depicts a flowchart of an example method of operation of a blood metrics measurement apparatus according to some embodiments.

FIG. 7 depicts a block diagram of an example blood pressure calculation system according to some embodiments.

FIG. 8 depicts a flowchart of an example method of channel selection according to some embodiments

FIG. 9 depicts a graph of example signal channels including an example pair of signal channels selected for calculation of modified pulse transit time.

FIG. 10 depicts a flowchart of an example method of calculating blood pressure according to some embodiments.

FIG. 11 depicts a flowchart of an example heart rate phase method according to some embodiments.

FIG. 12 depicts a flowchart of an example linear shift method according to some embodiments.

FIG. 13 is a block diagram illustrating an example digital device that can be utilized in the implementation of various embodiments.

DETAILED DESCRIPTION

Biometrics including blood metrics may be measured by minimally invasive procedures to address medical conditions such as diabetes or in the diagnosis and discovery of diseases. Minimal-invasive procedure based devices may have the advantages of reducing costs and decreasing the need for invasive methods, thereby increasing the comfort and well-being of users and patients. Even though these devices have revolutionized patient care, they have only been described in, and approved for, medical purposes. Minimal-invasive procedure based devices are usually out of reach for the general public because they are designed for medical uses rather than non-medical purposes such as fitness, well-being, and quality of life.

Personal devices such as sphygmomanometers or pulse oximeters measure blood pressure or oxygen levels, respectively, on a per-request basis. They usually cannot measure blood metrics real time or periodically. Real-time blood metrics data (e.g., high resolution measurements, or measurements over long periods of time) may allow these devices to facilitate users monitoring and controlling their energy levels and/or metabolism. Nutritionists, people suffering from obesity, people desiring to eat healthier, fitness enthusiasts, semiprofessional athletes, people likely to have hypoglycemia, or the vast majority of the general population can benefit from these devices.

In various embodiments, a multispectral blood metric measurement apparatus monitors blood metrics, fitness, and/or metabolism levels of various users in a non-invasive manner. The multispectral blood metric measurement apparatus may be, for example, wearable technology. The multispectral blood metric measurement apparatus may measure any number of blood metrics. Blood metrics may include, for example, various nutrient blood concentrations. Blood metrics may be, for example, monitored, stored, tracked, and/or analyzed.

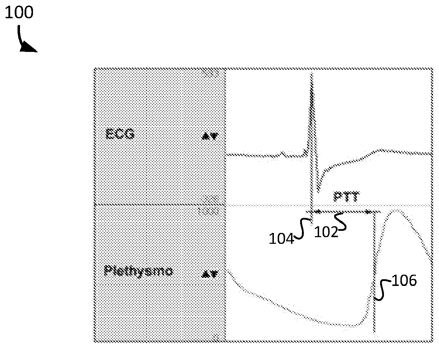

FIG. 1 depicts a graph 100 showing an example pulse transit time (or, "PTT") 102 according to some embodiments. Generally, pulse transit time is a measurement related to blood pressure, and may be used to non-invasively measure blood pressure using cuff-less approaches. Pulse transit time may correspond to a time taken for a cardiac pulse pressure wavefront to propagate along a user's arteries. This pressure wave may arise from an ejection of blood from the left ventricle. In one example, measurement of pulse transit time has been made by measuring the time difference between an R-wave on an ECG signal, and a peak on a photoplethysmogram (or, "PPG") wave on the fingertip of a user. FIG. 1 shows a variation of such an example - namely, a time taken 102 for the R-wave on an ECG signal 104 to a peak 106 in a derivative of the PPG wave at the fingertip of a user.

Pulse wave velocity (PWV) measured from pulse transit time (e.g., as distance traversed over the transit time) may be correlated with blood pressure. While pulse transit time alone may not be a reliable predictor of blood pressure, its use along with other features extracted from a PPG signal may provide a reliable estimate or calculation.

Related techniques use two PPG systems (e.g., an LED and photodetector pair) on the same appendage of a user. Such a system may not require the use of ECG signal, but instead may measure the transit time between the PPG waves as measured on each sub-system (e.g., LED-photodetector pair). This configuration may include placement of each of the two LEDs at different placement sites, e.g. wrist and digit, or elbow and wrist, and the like.

The difficulty with these techniques is that the distance between placement sites may not always the same. Additionally, equipping users with this configuration may be complicated and tedious for use in continuous monitoring where it may be expected that users are performing other tasks simultaneously.

Various embodiments described herein provide systems and methods including optical sensors (e.g., LEDs and photodiodes) at a single measurement site (e.g., wrist) using two or more optical systems (e.g., LED-Photodiode systems). In the example of two LED-Photodiode pairs, the two pairs may be spatially separated by a predetermined (or, "fixed") distance (e.g., ~1.5 cm), and the transit time between key feature points on the waves acquired from each of the two systems may be measured. The measured transmit time may be referred to as a difference in phase, (e.g., transit time as PTT-Modified or modified pulse transit time).

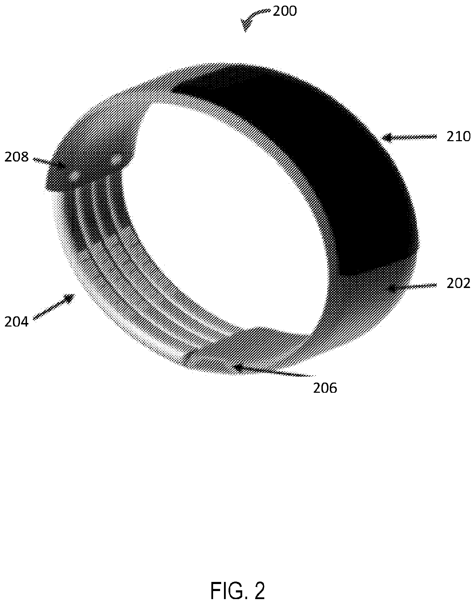

FIG. 2 depicts an example blood metrics measurement apparatus 200 according to some embodiments. The apparatus 200 comprises a central unit 202, a sensor array 204, and a coupling means 208. The central unit 202 may be a wearable member made of elastic and/or flexible hypoallergenic wearable material.

In the illustrated example, the sensor array 204 is coupled to the central unit 202. The sensor array 204 may comprise any number of energy transmitters and/or energy receivers. The sensor array 204 may be detached from the central unit 202. In some embodiments, the sensor array 202 may be mechanically and electrically coupled to the central unit 202. The sensor array 204 comprises various illumination (e.g., near infra-red, infra-red, or short infrared) and sensing array. The sensor array 204 may further comprise conductivity and/or capacity sensors. Different sensor array 204 may be provided to measure different blood metrics.

The central unit 202 may comprise an analyzer. In some embodiments, the central unit comprises an analyzer, one or more energy transmitter(s), and one or more energy receiver(s). The central unit 202 may further comprise a communication module and/or a battery compartment. The coupling means 208 are mounting screw holes in FIG. 2, however, it will be appreciated that coupling means may be optional. Further, coupling means 208 may include any kind of means including a clip, hook, switch, expanding fabric, adhesive, or the like. One of ordinary skill in the art would understand that other mounting means may be used.

The apparatus 200 further comprises a micro-USB port 206 to allow for communication with a digital device and a screen 210. Various user interfaces (e.g., lights, a display, touchscreen, or the like) may be displayed on the screen 210.

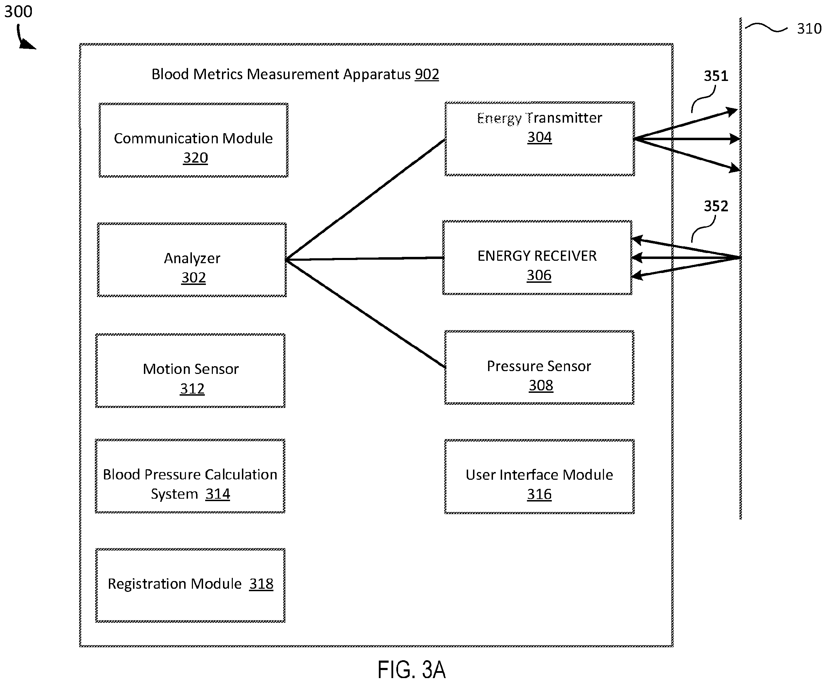

FIG. 3A depicts a block diagram 300 of an example blood metrics measurement apparatus 200 according to some embodiments. Generally, the blood metrics measurement apparatus 200 may be configured to facilitate non-invasive measurement of a user's blood pressure. In some embodiments, more particularly, the blood metrics measurement apparatus 200 facilitates non-invasive continuous measurement of a user's blood pressure. It will be appreciated that non-invasive continuous measurement may include measuring arterial blood pressure in real-time without interruption (e.g., without having to inflate and deflate a cuff) and without inserting a device (e.g., a tube or catheter) into to the user's tissue or body.

In some embodiments, the blood metrics measurement apparatus 200 may project energy into tissue of a user (e.g., the wearer of the apparatus 200) and detect (or, "receive") energy reflected from and/or transmitted through tissue of the user. In some embodiments, the blood metrics measurement apparatus 200 may project energy at one or more wavelengths (e.g., 523nm, 590nm, 623nm, 660nm, 740nm, 850nm, 940nm, etc.) from multiple light sources (e.g., light-emitting diodes). The detected energy may be a fraction (or, "portion") of the energy that is projected into the tissue. Energy at different wavelengths may be absorbed at a different rate that is related to a user's body state. The user's body state (e.g., heart rate, blood pressure, or the like) may determine the amount of absorbed energy. Accordingly, energy at different wavelengths may be absorbed at different levels by a user's body. The fraction of energy received (e.g., that is reflected by the tissue or transmitted through the tissue) may be used to generate signals, such PPG signals, at different levels. These signals may provide information of the user's body state. This information may be obtained by analyzing waveforms of the signal in a time domain and/or a frequency domain.

In some embodiments, the blood metrics measurement apparatus 200 may measure non-optical signals. For example, the blood metrics measurement apparatus 200 may be configured to non-invasively detect arterial pressure of one or more arteries of the user (e.g., radial artery or ulnar artery) based on pressure signals. Similar to optical signals, the pressure signals may be measured to provide information of the user's body state, and this information may be obtained by analyzing waveforms of the signal in a time domain and/or a frequency domain.

Functionality of the systems and modules described herein may be performed similarly with respect to both optical signals (e.g., PPG signals) and non-optical signals (e.g., pressure signals). Accordingly, it will be appreciated that signals, as used herein, may include optical signals, non-optical signals, or both.

In some embodiments, a user may comfortably wear the blood metrics measurement apparatus 200 over time. For example, the blood metrics measurement apparatus 200 may be worn without interrupting typical user activity (e.g., moving, walking, running, sleeping, etc.). The blood metrics measurement apparatus 200 may comprise lightweight components. The blood metrics measurement apparatus 200 may be made of hypoallergenic materials. The blood metrics measurement apparatus 200 may be flexibly built so that it may fit various body parts (e.g., wrist, earlobe, ankle, or chest) of a user.

In some embodiments, the blood metrics measurement apparatus 200 is capable of executing applications related to measuring blood metrics, such as blood pressure calculation, presenting a user interface through a display and/or communicating with various entities (e.g., mobile devices and/or other user devices) through a communication network. For example, the blood metrics measurement apparatus 200 may receive one or more blood metric measurements (e.g., one or more signals), track and store the blood metric measurements, analyze the blood metric measurements, and/or provide recommendations and/or messages based on the blood metric measurements. An application user interface may facilitate interaction between a user of the blood metrics measurement apparatus 200 and an application running on the blood metrics measurement apparatus 200.

In various embodiments, the blood metrics measurement apparatus 200 may perform analysis of the measurements (e.g., calculate blood pressure values), display results, provide reports, display progress, display historic readings, track measurements, track analysis, provide alerts (or, messages), and/or the like.

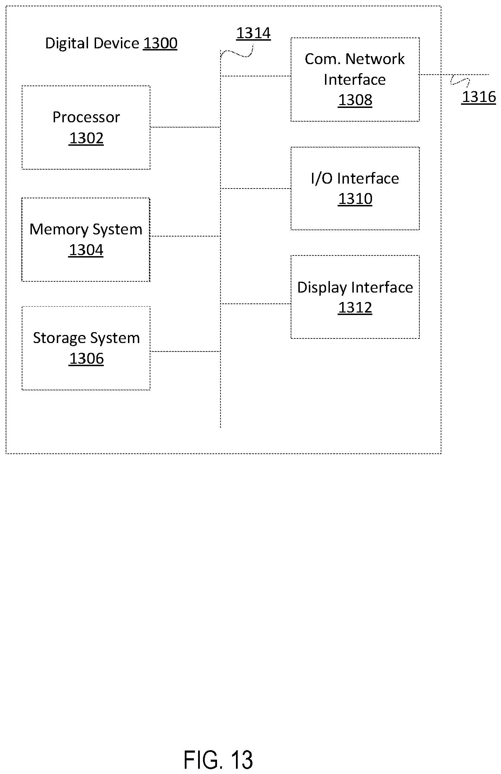

As used in this paper, computing devices (e.g., digital devices) may include a mobile phone, a tablet computing device, a laptop, a desktop computer, personal digital assistant, a portable gaming unit, a wired gaming unit, a thin client, a set-top box, a portable multi-media player, or any other type of network accessible user device known to those of skill in the art. The blood metrics measurement apparatus 200 may be implemented using one or more digital devices. An example digital device is described in FIG. 13.

In the example of FIG. 3A, the blood metrics measurement apparatus 200 comprises an analyzer 302, an energy transmitter 304, an energy receiver 306, a pressure sensor 308, a motion sensor 312, a blood pressure calculation system 314, a user interface module 316, a registration module 318, and a communication module 320. Various embodiments may comprise a wearable member. The wearable member may include, for example, a bracelet, glasses, necklace, ring, anklet, belt, broach, jewelry, clothing, or any other member of combination of members that allow the blood metrics measurement apparatus 200 to be close to or touch a body of the wearer. In some embodiments, the blood metrics measurement apparatus 200 may further comprise a driver (not shown) and a power source (not shown). The power source may be coupled to the energy transmitter 304 via the driver. The blood metrics measurement apparatus 200 may further comprise an Analog-to-Digital Converter ("ADC") (not shown). The ADC may be coupled to the energy receiver 306 and the analyzer 302.

The analyzer 302 may be coupled to the energy transmitter 304, the energy receiver 306, the pressure sensor 308, the motion sensor 312, and the communication module 314. The energy transmitter 304 and the energy receiver 306 may be secured to the wearable member such that the energy transmitter 304 and the energy receiver 306 may make contact or be in proximity with tissues (e.g., skin) of a user. In various embodiments, the energy transmitter 304 emits energy including, but not limited to, light, into the body of the user. The energy produced by the energy transmitter may be in the direction of entering tissues. For example, the energy produced by the energy transmitter 304 is in a direction 351 entering the tissue 310. In some embodiments, the energy transmitter 304 emits energy or light at different wavelengths. The energy transmitter 304 may comprise any number of light emission diodes ("LEDs"). In some embodiments, the energy transmitter 304 comprises at least two LEDs. Each LED may be configured to emit energy at one or more wavelengths. In another example, each LED may emit light with a peak wavelength centered around a wavelength. In one example, the energy transmitter 304 may emit light with a peak wavelength centered around 500 nm to 1800 nm, although the wavelength may include a variety of spectrums (e.g., IR, near-IR, and the like).

Each wavelength may correspond to one or more blood metrics of interest and/or one or more nutrients. It will be appreciated that different components of the blood and/or different nutrients may absorb energy at different wavelengths. In various embodiments, a controller, driver, analyzer 302, or the like may receive a blood metric or nutrient of interest (e.g., from a user of the blood metrics measurement apparatus 200 and/or a user device not shown). The controller, driver, analyzer 302 or the like may associate the blood metric and/or nutrient of interest with one or more wavelengths and configure one or more of the LEDs to emit energy of at least one of the one or more wavelengths. For example, the analyzer 302 may command the driver to deliver electric power to one LED that is configured to emit light at the desired wavelength.

The energy receiver 306 may detect energy associated with the energy provided by the LEDs from tissues (e.g., skin) of the user. In this example, received and/or detected energy is in the direction 352 that leaves from the tissue 310. In various embodiments, the energy receiver 306 may detect energy from the body of the user that is a fraction of the energy produced by the energy transmitter 304.

The energy transmitter 304 and the energy receiver 306 may be configured such that the energy receiver 306 detects reflected energy from tissues of the user of the multispectral blood metrics measurement apparatus 200. For example, the energy transmitter 304 and the energy receiver 306 may be configured to be disposed on one surface or side of a user's tissue. The energy transmitter 304 and the energy receiver 306 may be configured such that the energy receiver 306 detects energy from the energy transmitter 304 that passes through or reflects from the user's tissues. In some embodiments, the energy transmitter 304 and the energy receiver 306 may be configured to be disposed on different (e.g., opposite) surfaces or sides of a users' tissue.

The energy transmitter 304 may be configured to generate energy at a set of wavelengths. In some embodiments, the energy transmitter 304 is configured to generate energy such that energy at different wavelengths is generated sequentially and/or periodically. The energy transmitter 304 may be configured to generate energy at each particular wavelength until energy at all wavelengths of a set is generated. The period of time for the energy transmitter 304 to generate energy at all wavelengths is a generation period. Subsequent to completion of the generation period, the energy transmitter 304 may start a new generation period thereby allowing multiple measurements.

Energy detected from tissues of a user may be detected by the energy receiver 306. The energy receiver 306 may be configured to generate a signal in response to the detected energy. In some embodiments, the energy receiver 306 may be triggered by the energy received to generate an output which may be dependent or partially dependent upon the amount of energy received. The energy receiver 306 may be configured to generate a signal (e.g., an electric current, or an electric voltage) in response to the energy received from the tissues.

The signal generated by the energy receiver 306 may be associated with one or more blood metrics and/or nutrients of interest. Energy at different wavelengths may be absorbed at a different rate that is related to a user's body state. The user's body state (e.g., heart rate, blood pressure, nutrient level, or the like) may determine the amount of energy absorbed by the body. Accordingly, energy from the user's body at different wavelengths may be detected at different levels thereby causing different responses of the energy receiver 306. The energy receiver 306 may, for example, output signals based on the level of the energy received.

The energy receiver 306 may provide information associated with the user's body state. Blood metric information may be determined (e.g., by the analyzer 302) from the output signal of the energy receiver 306. In some embodiments, the energy receiver 306 may comprise a set of photodetectors (e.g., a photo diode, or a photo transistor) which are configured to output a signal dependent upon photons or the like from the energy transmitter 304 that passed through tissues of the user.

In various embodiments, the output signal of the energy receiver 306 is a composite of multiple signals. Each signal of the composite may be associated with energy at a wavelength which may be a portion (or fraction) of the total energy emitted by the energy transmitter 304.

The pressure sensor 308 may be configured to generate, detect, and/or measure non-optical signals. For example, the pressure sensor 308 may non-invasively and continuously generate, detect and/or measure pressure pulse signals. In some embodiments, the pressure sensor 308 measures pressure pulse waveforms associated with arterial pressure of one or more arteries of a user. In various embodiments, the blood metrics measurement apparatus 200 may include the energy transmitter 304 to generate optical signals and the energy receiver 306 to receive optical signals but not the pressure sensor 308. Alternately, the blood metrics measurement apparatus 200 may include the pressure sensor 308 that may produce pressure on the user's body and/or receive measurements based on that pressure but not the energy transmitter 304 or the energy receiver 306 to receive optical signals.

In some embodiments, the motion sensor 312 may be configured to detect position and orientation of the blood metrics measurement apparatus 200, and detect motion of the blood metrics measurement apparatus 200. For example, the blood metrics measurement apparatus 200 may detect position, orientation, and motion along an x, y, or z-axis, and measured values may include velocity, acceleration, distance, and the like. In some embodiments, the motion sensor 312 may include one or more accelerometers, gyroscope, global positioning systems, or the like. The motion sensor may be coupled to the analyzer 302.

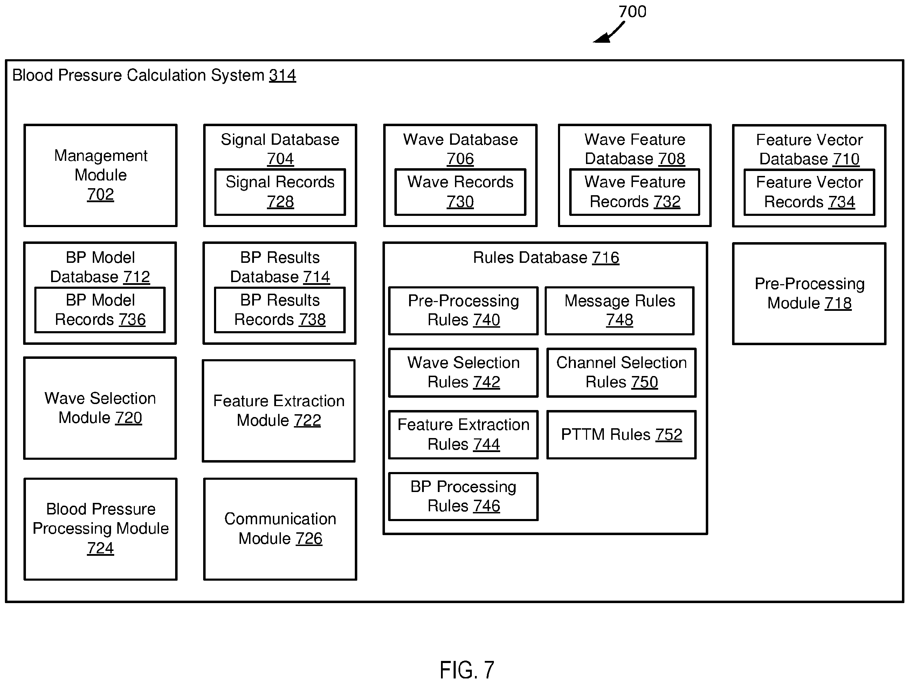

The blood pressure calculation system 314 may be configured to calculate blood pressure values (e.g., systolic, diastolic), and generate messages or alerts based on those values. An example of the blood pressure calculation system 314 is discussed further below with reference to FIG. 7.

The user interface module 316 may be configured to present images, audio, haptics, and the like, corresponding to health data, such as blood pressure values, messages, alerts, and so forth. For example, the user interface module 316 may display one or more graphical user interfaces (GUIs) to present a calculated blood pressure to a user.

The registration module 318 may be configured to generate registration requests to create, read, update, delete, or otherwise access, registration records associated with user accounts (e.g., a user account associated with a user of the blood metrics measurement apparatus 200) and registration records associated with the blood metrics measurement apparatus 200. In some embodiments, a user inputs user account registration information and blood metrics measurement apparatus registration information via the user interface module 316. For example, user account registration information may include geographic attributes, demographic attributes, psychographic attributes, and/or behavioristic attributes. Accordingly, user account registration information may include some or all of the following attributes:

- User Account Identifier: Identifier that identifies a user account.

- Password: Password, or other personal identifier, used to authenticate the user account. For example, it may an alphanumerical password, biometric data (e.g., fingerprint, etc.). In some embodiments, readings or measurements from the blood metrics measurement apparatus 200 may be used to authenticate the user account.

- Device Identifier(s): Identifier(s) that identify one or more blood metric measurement apparatus' associated with the user account.

- Name: A name of the user.

- DOB: A date of birth of the user.

- Age: An age of the user.

- Gender: Gender of the user (e.g., female, male, transgender, or the like).

- Weight: A weight of the user.

- Height: A height of the user.

- Skin color: A skin color of the user.

- Activity Level: An activity level of the user (e.g., sedentary, lightly active, active, very active, and so forth).

- Geographic location: A location of the user (e.g., as determined by a location service and/or specified by the user).

- Blood Pressure Profile: Hypertensive, Hypotensive, Normal or unknown.

- Blood Glucose Profile (e.g., Diabetes information)

- Wrist circumference: Circumference of the user's wrist.

In some embodiments, the blood metrics measurement apparatus registration information includes some or all of the following attributes:

- Apparatus Identifier: Identifier that identifies a blood metrics measurement apparatus.

- User Account Identifier: Identifier that identifies a user account associated with the blood metrics measurement apparatus.

- Geographic location: A current location of the blood metrics measurement apparatus (e.g., as determined by a location service and/or specified by the user).

- Settings: One or more settings of the blood measurement metrics apparatus. For example, some or all of the settings may be automatically determined based on one or more user account attributes (e.g., height, weight, or the like) and/or by the user.

The communication module 320 may be configured to send requests to and receive data from one or a plurality of systems. The communication module 320 may send requests to and receive data from a systems through a network or a portion of a network. Depending upon implementation-specific or other considerations, the communication module 320 may send requests and receive data through a connection, all or a portion of which may be a wireless connection. The communication module 320 may request and receive messages, and/or other communications from associated systems.

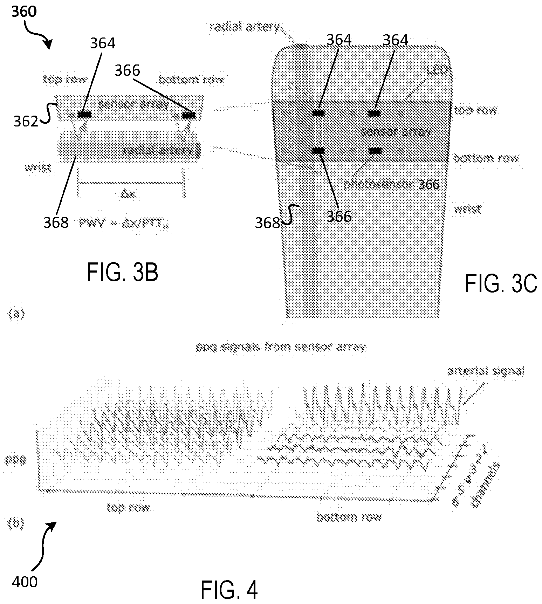

FIG. 3B depicts a block diagram of an example sensor system 360 according to some embodiments. In some embodiments, the sensor system 360 may be disposed in the blood metrics measurement apparatus 200. For example, the energy transmitter 304 and the energy receiver 306 may comprise the sensor system 360. The sensor system 360 may include an optical sensor array 362 including one or more energy transmitters 364 (e.g., LEDs or other lights sources) and one or more corresponding energy receivers 366 (e.g., photodiodes or other photosensors). It will be appreciated that each pair of corresponding energy transmitters and energy receivers (e.g., energy transmitter 364 and energy receiver 366 as well as energy may be referred to as an LED-PD system, and the sensor system 360 may include any number of such LED-PD systems.

In some embodiments, the energy transmitters 364 each comprise a set of LEDs (e.g., between 2-6 LEDs) configured to transmit a variety of wavelengths into tissue of a user. The corresponding energy receivers 366 may each comprise one or more photodiodes which receive returning light from the corresponding energy transmitters 364 after passage through tissue. In some embodiments, the energy transmitters 364 and the energy receivers 366 may be spaced at a predetermined distance (e.g., 15 mm) from each other.

In some embodiments, the sensor system 360 may be mounted on a user's wrist along the radial artery 368, or other area of the user's body along arterial pathways (e.g., ulnar artery). The sensor system 360 may include an accelerometer and gyroscope for detecting position and orientation information.

In some embodiments, the sensor system 360 may include a pressure sensor. For example, at least one LED within each LED-PD system may be configured to sample data from tissue at a very high frequency (upwards of 1 KHz), and the other LEDs may sample at a lower rate (around 50-100 Hz). The channels sampling data at high frequency may be referred to as the fast LED channels, and the channels sampling data at lower frequency may be referred to as slow LED channels.

The velocity of blood moving through the arteries may vary within a range of 5 m/s to 15 m/s. This velocity may be correlated with various factors (e.g., age, arterial stiffness, blood pressure, some of which may be interrelated). In PPG measurement devices, different sensor location may cause a significant difference in signal characteristics. This may, for example, prevent PPG sensors from receiving a high quality signal that enables high resolution analysis in time and frequency domains. For example, the signal shape of capillary tissue, venous tissue and of arterial tissue may be inherently different. This may signify that a 5mm translation may be the difference between an arterial signal and a signal that is practically unusable, and even simple measurements (e.g., heart rate measurement) becomes difficult.

In order to obtain an arterial signal with a higher degree of flexibility in device placement, the blood metrics measurement apparatus 200 uses an array of LED/photodiode pairs (or, "channels") that facilitates measurement of PPG signals at any number of locations (e.g., 12 different locations). FIG. 3B shows an example configuration of an LED-PD sensor array used in the blood metrics apparatus 200. More specifically, FIG. 3B shows a sideways profile of the blood metrics apparatus 200 mounted along the direction of the radial artery of a user. FIG. 3C shows an example configuration of sensors 364 and 366 mounted on the left wrist (facing the palm) of a user.

In the example of FIGS. 3B and 3C, the LED-PD pairs 364 and 366 are separated by a known fixed distance (e.g., 15mm), which allows a pulse wave velocity (or "PWV") to be derived from a modified pulse transit time. FIG. 4 shows an example of a PPG time series data collected from the sensor array 362. For example, the sensor array 360 may comprise twelve sensors (e.g., six channels comprising six LED-PD pairs).

The pairs of LED-PD systems or channels may operate at a high sampling rate, e.g. 1.4 KHz, where the LEDs are activated at this sampling rate, and the photodiode measures the light returned from tissue. In one example, twelve PPG time series are generated in this manner, one from each LED-photodiode pair. This sampling rate may be determined based on the following: in order to be able to resolve a 3mms time shift, one needs a signal of at least about 700 Hz. To avoid introducing error when sampling that signal, the sampling rate is set at the nyquist frequency for that signal, which corresponds to 1.4 KHz.

FIG. 4 depicts a graph 400 of example waves from each LED-PD pair for six signal channels. LED-PD pairs placed directly on top of the artery show a strong arterial signals and are strongly correlated (channels 1 in the top and bottom row), while others away from the artery have much lower signal to noise ratio.

The PPG time series generally corresponding to arterial signal appear similar in shape to each other, but a small phase shift separates the time series from the top row and the bottom row of sensor on the device. This small phase shift is introduced by the small delay it takes the pressure wavefront to travel from the measurement site at the bottom row of sensor to the top row, and corresponds to the modified pulse transit time.

In various embodiments, in order to calculate modified pulse transit time, a pair of channels may be selected that correspond to an arterial signal. Channel selection and modified pulse transit time are discussed further below.

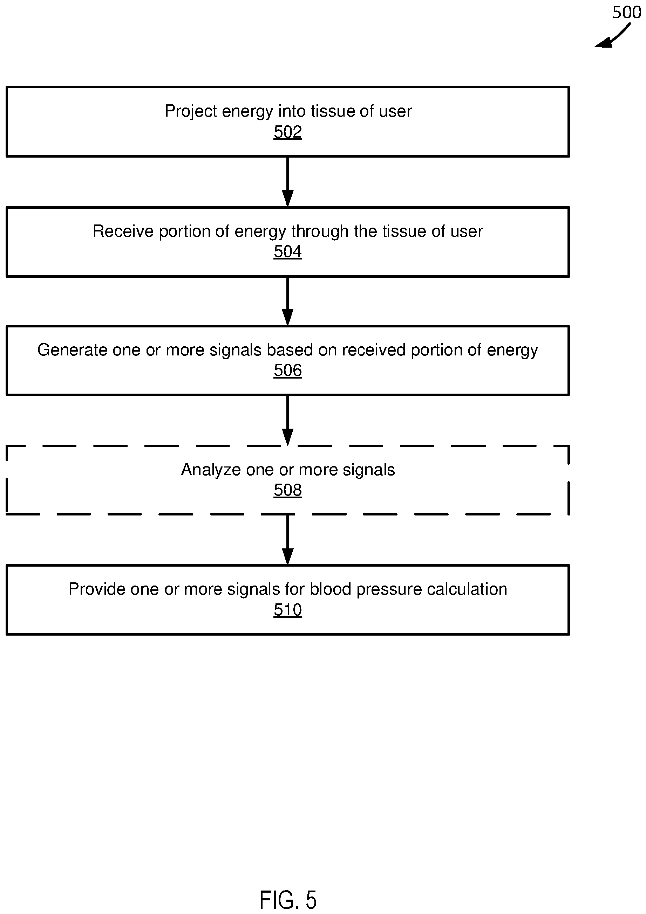

FIG. 5 depicts a flowchart 500 of an example method of operation of a blood metrics measurement apparatus (e.g., blood metrics measurement apparatus 200) according to some embodiments. In this and other flowcharts described herein, the flowchart illustrates by way of example a sequence of steps. It should be understood the steps may be reorganized for parallel execution, or reordered, as applicable. Moreover, some steps that could have been included may have been removed to avoid providing too much information for the sake of clarity and some steps that were included could be removed, but may have been included for the sake of illustrative clarity.

In step 502, a blood metrics measurement apparatus projects energy into tissue of a user (e.g., the user wearing blood metrics measurement apparatus). The energy may be projected from a transmitter (e.g., energy transmitter 304) comprising a plurality of light sources (e.g., LEDs). In some embodiments, a first light source (e.g., one or more LEDs) may project light energy at a plurality of different wavelengths, such as 523nm, 590nm, 623nm, 660nm, 740nm, 850nm, and 940nm, and a second light source (e.g., one or more LEDs) may project energy at the same, or substantially similar, wavelength as one of the wavelengths projected by the first light source (e.g., 523nm, 590nm, 623nm, 660nm, 740nm, 850nm, or 940nm). It will be appreciated that other configuration may be used (e.g., a greater number of light sources) a greater or lesser number of wavelengths projected from the lights sources, and so forth.

In step 504, the blood metrics measurement apparatus receives (or, "detects) portions of energy through the tissue of the user. In some embodiments, an energy receiver (e.g., energy transmitter 306) detects a portion of the energy transmitted into the user's tissue by the energy transmitter. The energy receiver may generate a signal based on the portion of energy detected (e.g., based on the amount of the energy detected). For example, energy detected may be a portion of the energy projected at step 502 reflected by the tissue. By way of further example, energy detected may be a portion of the energy projected at step 502 that passes through the tissue (e.g., other undetected energy may be absorbed by tissue and/or otherwise blocked). In various embodiments, steps 502 and 504 are performed simultaneously or substantially simultaneously. That is, energy projection and detection may be performed approximately simultaneously.

In step 506, the blood metrics measurement apparatus generates one or more signals based on the received portions of energy. In some embodiments, the energy receiver may generate a multi-channel PPG signal (e.g., as mentioned above). The output (or, "generated") signal of the energy receiver may be an electric current or an electric voltage, of which the amplitude may be related to the amount of the energy detected.

In various embodiments, analysis of the signals from the energy receiver may identify abnormal measurements. For example, each of the measurements may be compared to a predetermined value. If the difference between the measurement and the predetermined value is above (or below) a threshold, then the measurement may be determined to be abnormal. An abnormal value may trigger additional analysis or an alert. In some embodiments, an abnormal value is ignored (e.g., as possibly effected by noise caused by movement of the energy transmitter and/or the energy receiver). In various embodiments, the abnormal value may be discounted (e.g., the weight of the value reduced). The degree of discount may be based, for example, on information from an accelerometer (e.g., a large acceleration may indicate that the abnormal value should be significantly discounted) and/or based on historical values. It will be appreciated that the degree of discount may be based on any number of factors.

In some embodiments, measurements may be averaged over a period of time. A Kalman filer (e.g., a nonlinear, unscented Kalman filter) may be applied to any number of measurements or averaged measurements. A motion measurement (e.g., a measurement by an accelerometer) may be considered. Upon determining a measurement is abnormal, the motion measurement for that time point may be inspected. A large measurement may indicate large vibrations or accelerations that corroborate that the measurement may be abnormal. Measurements collected in such situations are likely to have significant electrical noises.

At step 508, the blood metrics measurement apparatus analyzes signals from the energy receiver analyzed in the frequency domain to determine blood metrics. Concentration of a nutrient in the blood may subsequently be determined. In some embodiments, signals may be provided to a bandpass filter that separates AC components from DC components. An AC component may represent signal variation at the cardiac frequency and a DC component may represent the average overall transmitted light intensity. In some embodiments, a heart rate and/or oxygen saturation, $SpO_2$ may be determined. The heart rate may be determined, for example, by averaging the maximum frequency to determine the rate of cardiac beats in a predetermined amount of time. The oxygen saturation $SpO_2$ may be determined according to Equation (1):

$$S_pO_2 = 110 - 25 \times R$$

$$(1),$$

where R is the ration of a red and infrared normalized transmitted light intensity. R may be determined according to Equation (2):

$$R \approx \frac{AC_R/DC_R}{AC_{IR}/DC_{IR}}$$

$$(2),$$

where the $AC_R$ is the AC component of the detected energy corresponding to a wavelength (e.g., red light), $DC_R$ is the DC component of the detected energy corresponding to the wavelength (e.g., red light), $AC_{IR}$ is the AC component of the detected energy corresponding to a different wavelength (e.g., infrared light), and $DC_{IR}$ is the DC component of the detected energy corresponding to the different wavelength (e.g., infrared light). In some embodiments, the AC component may be selected as the highest spectral line in the cardiac frequency band. Waveform analysis may be performed to determine the R-R interval defined by two successive AC components, an elapsed interval and the probation, if there is any.

It will be appreciated that analysis may be performed by the analyzer and/or any other digital device (e.g., user device).

State space estimation and progression may be performed to determine blood metrics. A system may be modeled according to Equation (3):

$$x(n + 1) = f[x(n)] + u(n)$$
$$y(n) = h[x(n)] + v(n)$$

$$(3),$$

where x(n) represents the state of the system, u(n) is process noise, y(n) is the vector of the observed signals, and v(n) is the measurement noise.

Table 1 lists one or more parameters for x(n) as well as their initial value in some embodiments:

Table 1

| Parameter | Symbol | Initial Value |
|---|---|---|
| Cardiac frequency | $f_{HR}$ | 1 *Hz* |
| Cardiac phase | $\theta_{HR}$ | 0 |
| Cardiac harmonic amplitude | $I^{HR}_{Harmonic}$ | 0 |
| Cardiac Pulse Pressure | $P_{HR}$ | 1 |
| Point Blood Pressure | $P_{Point}$ | 1 |
| Respiratory frequency | $f_{Resp}$ | 0.3 *Hz* |
| Respiratory phase | $\theta_{Resp}$ | 0 |
| Wavelength i=1..N AC peak amplitude | $I^{AC}_{\lambda_i}$ | 0.5 max_value |
| Wavelength i=1..N AC peak location | $pos^{AC}_{\lambda_i}$ | Corresponding FFT bin to 1 *Hz* |
| Wavelength i=1..N DC | $I^{DC}_{\lambda_i}$ | 0.5 max_value |
| Wavelength i=1..N p2p amplitude | $I^{p2p}_{\lambda_i}$ | 1 ADC read |
| Wavelength i=1..N rise time | $\tau^{rise}_{\lambda_i}$ | 0.1 sec |
| Wavelength i=1..N Significance coefficient | $c_{\lambda_i}$ | 1 |

(continued)

| Parameter | Symbol | Initial Value |
|---|---|---|
| Wavelength i=1..N HRV | $T_{\lambda_i}^{HRV}$ | 1 sec |
| Best Ratio pH | $BR_{pH}$ | 2 |
| Best Ratio pCO2 | $BR_{pCO2}$ | 3 |
| Best Ratio pHCO3- | $BR_{pHC03}^-$ | 4 |
| Acceleration magnitude | $I_{move}$ | 0 |
| GPS velocity | $|v|_{GPS}$ | 0 |
| GPS altitude | $|alt|_{GPS}$ | 0 |
| GPS acceleration | $|a|_{GPS}$ | 0 |
| GPS incline | $|incline|_{GPS}$ | 0 |
| Restfulness | $Rest$ | 0 |
| Hydration | $Hyd$ | 0 |
| Systolic Blood Pressure | SBP | 120 mmHg |
| Diastolic Blood Pressure | DBP | 80 mmHg |
| End tidal CO2 | ETCO2 | 40 mmHg |
| Blood Carbon Monoxide | SpCO | 0% |

Table 2 lists one or more parameters for y(n) as well as their initial value in some embodiments:

Table 2

| Parameter | Symbol | Initial |
|---|---|---|
| Blood pH | $pH$ | 7.35 |
| Blood PCO2 | $pCO_2$ | 24 $mmol$ |
| Blood PO2 | $pO_2$ | 24 $mmol$ |
| Blood PHCO3- | $pHCO_3^-$ | 24 $mmol$ |
| Blood Glucose | $pC_6H_{12}O_6$ | 3 $mmol$ |
| Cardiac Frequency | $f_{HR}$ | 1 |
| Point Blood Pressure | $P_{Point}$ | 1 |
| Respiratory Frequency | $f_{Resp}$ | 0.3 |
| GPS velocity | $|v|_{GPS}$ | 0 |
| GPS altitude | $|alt|_{GPS}$ | 0 |
| GPS acceleration | $|a|_{GPS}$ | 0 |
| GPS incline | $|incline|_{GPS}$ | 0 |

Table 3 lists the state space model F(X(n)) between the parameters listed in Table 1 and Table 2 in some embodiments, where the energy wavelengths comprise 880nm, 631nm, 1450nm, and 1550nm:

| Name | Symbol | Equation |
|---|---|---|
| Cardiac frequency | $f_{HR}$ | $$bin\_to\_freq\left(\frac{\Sigma c_{\lambda_i} pos_{\lambda_i}^{AC}}{\Sigma c_{\lambda_i}}\right)$$ |
| Cardiac phase | $\theta_{HR}$ | $\theta_{HR}(n-1) + f_s^{-1} * \omega^*,$ where $\omega^* \in [\omega\_\min, \omega\_\max]$ |
| Cardiac harmonic amplitude | $I_{Harmonic}^{HR}$ | $$\frac{\Sigma c_{\lambda_i} I_{\lambda_i}^{p2p}}{\Sigma c_{\lambda_i}}$$ |
| Cardiac Pulse Pressure | $P_{HR}$ | $\left(\frac{\Sigma c_{\lambda_i} \tau_{\lambda_i}^{rise}}{\Sigma c_{\lambda_i}}\right)^\wedge - 1$ |
| Point Blood Pressure | $P_{Point}$ | $\tau_{\lambda_1}^{rise^{-1}}$ |

| Name | Symbol | Equation |
|---|---|---|
| Respiratory frequency | $f_{Resp}$ | *3) Respiratory and Heart Rate State Models:* The fluctuations in the respiratory rate $\omega_r(n)$ and fluctuations in the heart rate $\omega_{ca}(n)$ that are not due to RSA are both modeled as a first-order autoregressive process with a mean and mild nonlinearity that limit the frequencies to know physiologic ranges $$\omega_r(n+1) = \bar{\omega}_r + \alpha_r\{s_r[\omega_r(n)] - \bar{\omega}_r\} + u_{\omega_r}(n) \quad (15)$$ $$\omega_{ca}(n+1) = \bar{\omega}_c + \alpha_c\{s_c[\omega_{ca}(n)] - \bar{\omega}_c\} + u_{\omega_{ca}}(n) \quad (16)$$ where $\bar{\omega}_r$ and $\bar{\omega}_c$ are the *a priori* estimates of the expected respiratory and cardiac frequencies, respectively; $\alpha_r$ and $\alpha_c$ control the bandwidth of the frequency fluctuations; and $u_{\omega_r}(n)$ and $u_{\omega_{ca}}(n)$ are white noise processes that model the random variation in the respiratory and cardiac frequencies, respectively. The instantaneous respiratory and heart rates in units of Hz are then $$f_r(n) = \frac{1}{2\pi T_s} s_r[\omega_r(n)] \quad (17)$$ $$f_c(n) = \frac{1}{2\pi T_s} s_c[\omega_c(n)]. \quad (18)$$ |
| Respiratory phase | $\theta_{Resp}$ | $\theta_{Resp}(n-1) + f_s^{-1} * \omega^*$, where $\omega^* \in [\omega\_min, \omega\_max]$ |
| $\lambda = 880$nm AC peak | $I_{\lambda_i}^{AC}$ | From FFT |
| $\lambda = 880$nm DC | $pos_{\lambda_i}^{AC}$ | From FFT |
| $\lambda = 880$nm p2p amplitude | $I_{\lambda_i}^{DC}$ | From Waveform analysis |
| $\lambda = 880$nm rise time | $I_{\lambda_i}^{p2p}$ | From Waveform analysis |
| $\lambda = 880$nm signal trend | $\tau_{\lambda_i}^{rise}$ | From Waveform analysis |
| $\lambda = 880$nm Significance coefficient | $c_{\lambda_i}$ | From Waveform analysis |
| $\lambda = 880$nm HRV | $T_{\lambda_i}^{HRV}$ | From Waveform analysis |
| $\lambda = 631$nm AC peak | $I_{\lambda_i}^{AC}$ | From Fast Fourier Transformation ("FFT") |
| $\lambda = 631$nm DC | $pos_{\lambda_i}^{AC}$ | From FFT |

| Name | Symbol | Equation |
|------|--------|----------|
| | | |
| $\lambda = 631\,\text{nm}$ p2p amplitude | $I_{\lambda_i}^{DC}$ | From Waveform analysis |
| $\lambda = 631\,\text{nm}$ rise time | $I_{\lambda_i}^{p2p}$ | From Waveform analysis |
| $\lambda = 631\,\text{nm}$ signal trend | $\tau_{\lambda_i}^{rise}$ | From Waveform analysis |
| $\lambda = 631\,\text{nm}$ Significance coefficient | $c_{\lambda_i}$ | From Waveform analysis |
| $\lambda = 631\,\text{nm}$ HRV | $T_{\lambda_i}^{HRV}$ | From Waveform analysis |
| $\lambda = 1450\,\text{nm}$ AC peak | $I_{\lambda_i}^{AC}$ | From FFT |
| $\lambda = 1450\,\text{nm}$ DC | $pos_{\lambda_i}^{AC}$ | From FFT |
| $\lambda = 1450\,\text{nm}$ p2p amplitude | $I_{\lambda_i}^{DC}$ | From Waveform analysis |
| $\lambda = 1450\,\text{nm}$ rise time | $I_{\lambda_i}^{p2p}$ | From Waveform analysis |
| $\lambda = 1450\,\text{nm}$ signal trend | $\tau_{\lambda_i}^{rise}$ | From Waveform analysis |
| $\lambda = 1450\,\text{nm}$ Significance coefficient | $c_{\lambda_i}$ | From Waveform analysis |
| $\lambda = 1450\,\text{nm}$ HRV | $T_{\lambda_i}^{HRV}$ | From Waveform analysis |
| $\lambda = 1550\,\text{nm}$ AC peak | $I_{\lambda_i}^{AC}$ | From FFT |
| $\lambda = 1550\,\text{nm}$ DC | $pos_{\lambda_i}^{AC}$ | From FFT |
| $\lambda = 1550\,\text{nm}$ p2p amplitude | $I_{\lambda_i}^{DC}$ | From Waveform analysis |
| $\lambda = 1550\,\text{nm}$ rise time | $I_{\lambda_i}^{p2p}$ | From Waveform analysis |
| $\lambda = 1550\,\text{nm}$ signal trend | $\tau_{\lambda_i}^{rise}$ | From Waveform analysis |
| $\lambda = 1550\,\text{nm}$ Significance coefficient | $c_{\lambda_i}$ | From Waveform analysis |

| Name | Symbol | Equation |
|------|--------|----------|
| $\lambda = 1550\text{nm}$ HRV | $T_{\lambda_i}^{HRV}$ | From Waveform analysis |
| Best Ratio pH | $BR_{pH}$ | Device Calibration |
| Best Ratio pCO2 | $BR_{pCO2}$ | Device Calibration |
| Best Ratio pHCO3- | $BR_{pHCO3^-}$ | Device Calibration |
| Acceleration magnitude | $I_{move}$ | From Accelerometer |
| GPS velocity | $|v|_{GPS}$ | From GPS |
| GPS altitude | $|alt|_{GPS}$ | From GPS |
| GPS acceleration | $|a|_{GPS}$ | From GPS |
| GPS incline | $|incline|_{GPS}$ | From GPS |

Table 3

Table 4 lists Y(n) = H(x(n)):

Table 4

| Name | Symbol | Equation |
|------|--------|----------|
| Blood pH | $pH$ | $$6.1 + \log(\frac{pHCO_3^-}{0.03pCO_2})$$ |
| Blood PCO2 | $pCO_2$ | $$\frac{\epsilon_{Hb}^{CO_2} - \epsilon_{Hb}^{Hb} * I_{\lambda_{CO_2}}^{AC} * I_{\lambda_1}^{DC}/(I_{\lambda_1}^{AC} * I_{\lambda_{CO_2}}^{DC})}{\epsilon_{Hb}^{CO_2} - \epsilon_{CO_2}^{CO_2} + \left(\epsilon_{CO_2}^{Hb} - \epsilon_{Hb}^{Hb}\right) * I_{\lambda_{CO_2}}^{AC} * I_{\lambda_1}^{DC}/(I_{\lambda_1}^{AC} * I_{\lambda_{CO_2}}^{DC})}$$ |
| Blood PO2 | $pO_2$ | $$\frac{\epsilon_{Hb}^{O_2} - \epsilon_{Hb}^{Hb} * I_{\lambda_{O_2}}^{AC} * I_{\lambda_1}^{DC}/(I_{\lambda_1}^{AC} * I_{\lambda_{O_2}}^{DC})}{\epsilon_{Hb}^{O_2} - \epsilon_{O_2}^{O_2} + \left(\epsilon_{O_2}^{Hb} - \epsilon_{Hb}^{Hb}\right) * I_{\lambda_{O_2}}^{AC} * I_{\lambda_1}^{DC}/(I_{\lambda_1}^{AC} * I_{\lambda_{O_2}}^{DC})}$$ |
| Blood PHCO3- | $pHCO_3^-$ | $$\frac{\epsilon_{Hb}^{HCO_3^-} - \epsilon_{Hb}^{Hb} * I_{\lambda_{HCO_3^-}}^{AC} * I_{\lambda_1}^{DC}/(I_{\lambda_1}^{AC} * I_{\lambda_{HCO_3^-}}^{DC})}{\epsilon_{Hb}^{HCO_3^-} - \epsilon_{HCO_3^-}^{HCO_3^-} + \left(\epsilon_{HCO_3^-}^{Hb} - \epsilon_{Hb}^{Hb}\right) * I_{\lambda_{HCO_3^-}}^{AC} * I_{\lambda_1}^{DC}/(I_{\lambda_1}^{AC} * I_{\lambda_{HCO_3^-}}^{DC})}$$ |
| Blood Glucose | $pC_6H_{12}O_6$ | As above |
| Cardiac Frequency | $JHR$ | As in f(x(n)) |
| Point Blood Pressure | $P_{Point}$ | As in f(x(n)) |
| Respiratory Frequency | $f_{Resp}$ | As in f(x(n)) |

(continued)

| Name | Symbol | Equation |
|------|--------|----------|
| GPS velocity | $|v|_{GPS}$ | As in f(x(n)) |
| GPS altitude | $|alt|_{GPS}$ | As in f(x(n)) |
| GPS acceleration | $|a|_{GPS}$ | As in f(x(n)) |
| GPS incline | $|incline|_{GPS}$ | As in f(x(n)) |

As illustrated in Tables 3 and 4, by generating energy at different wavelengths, one or more blood metrics may be determined from the detected energy. For example, cardiac frequency, cardiac phase, cardiac harmonic amplitude, cardiac pulse pressure, point blood pressure, respiratory frequency, respiratory phase, blood pH, blood $pCO_2$, blood $pHCO_3$., or blood glucose, may be determined.

In step 510, the blood metrics measurement apparatus provides the generated one or more signals for blood pressure calculation. In some embodiments, a communication module (e.g., communication module 320) provides the one or more signals (e.g., to a blood pressure calculation system and/or user device).

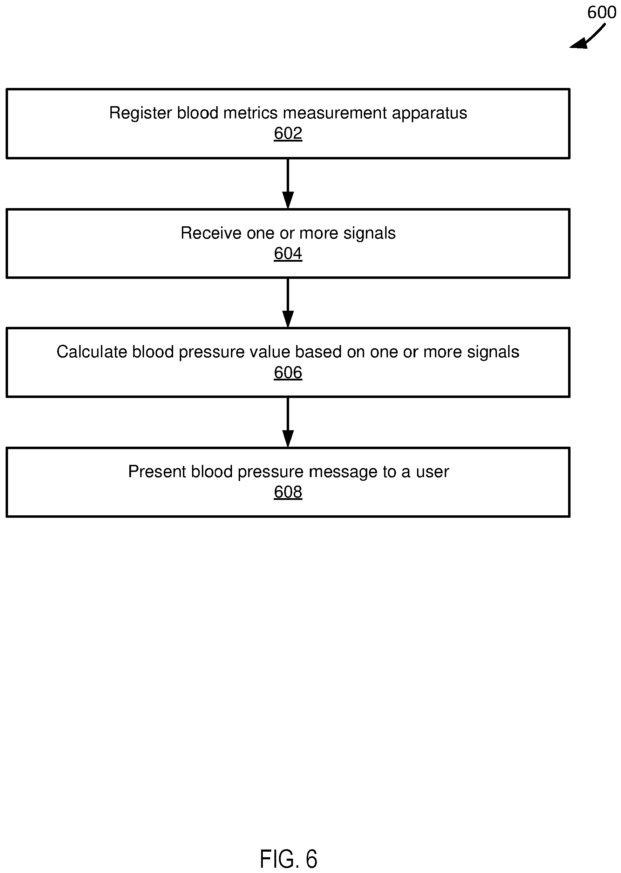

FIG. 6 depicts a flowchart 600 of an example method of operation of a blood metrics measurement apparatus (e.g. blood metrics measurement apparatus 200) according to some embodiments.

In step 602, a blood metrics measurement apparatus (e.g., blood metrics measurement apparatus 200) is registered. In some embodiments, input from a user is received by a user interface module (e.g., user interface module 316) that triggers a registration module (e.g., registration module 318) to generate a registration request to associate the blood metrics measurement apparatus with a user and/or user device. The registration request may include, for example, one or more blood metrics measurement apparatus attributes. In some embodiments, a communication module (e.g., communication module 320) provides the registration request to a server or other remote device.

In step 604, the blood metrics measurement apparatus receives one or more signals from the registered blood metrics measurement apparatus. In some embodiments, the one or more signals comprise optical signals (e.g., multi-channel PPG signals) and/or one or more non-optical signals (e.g., multi-channel pressure pulse signals). In some embodiments, the one or more signals may be received by the communication module.

In step 606, the blood metrics measurement apparatus calculates one or more arterial blood pressure values (e.g., systolic values and/or diastolic values) based on the received one or more signals. In some embodiments, a blood pressure calculation system (e.g., blood pressure calculation system 314) calculates the one or more arterial blood pressure values. Although this example depicts the blood metrics measurement apparatus calculating the one or more arterial blood pressure values, it will be appreciated that one or more other systems having the functionality of a blood pressure calculation system may perform the calculation. For example, in some embodiments, the blood metrics measurement apparatus and/or an associated user device may include such functionality and perform the calculation.

In step 608, the blood metrics measurement apparatus presents a blood pressure message to the user based on at least one of the one or more calculated arterial blood pressure values. For example, the message may include some or all of the arterial blood pressure values, alerts (e.g., high BP, low BP, good BP, poor BP, etc.) based on one or more of the calculated values, and so forth. In some embodiments, the blood metrics measurement apparatus presents (e.g., via images, audio, vibrations, etc.) the blood pressure message or alert to the user via the user interface module, or other feature of the blood metrics measurement apparatus.

FIG. 7 depicts a block diagram 700 of an example blood pressure calculation system 314 according to some embodiments. Generally, the blood pressure calculation system 314 may be configured to calculate arterial blood pressure values of a user. The blood pressure calculation system 314 may also store calculated arterial blood pressure values (e.g., for health tracking, etc.), and communicate with other systems. In some embodiments, the blood pressure calculation system 314 includes a management module 702, a signal database 704, a wave database 706, a wave feature database 708, a feature vector database 710, a blood pressure model database 712, a blood pressure results database 714, a rules database 716, a pre-processing module 718, a wave selection module 720, a feature extraction module 722, a blood pressure processing module 724, and a communication module 726.

The management module 702 may be configured to manage (e.g., create, read, update, delete, or access) signal records 728 stored in the signal database 704, wave records 730 stored in the wave database 706, wave feature records 732 stored in the wave feature database 708, feature vector records 734 stored in the feature vector database 710, empirical blood pressure model records 736 stored in the blood pressure model database 712, blood pressure result records 738 stored in the blood pressure results database 77, and/or rules 740 - 752 stored in rules database 716. The management module 702 may perform these operations manually (e.g., by an administrator interacting with a GUI) and/or automatically (e.g., by one or more of the modules 718 - 724). In some embodiments, the management module 702 comprises a library of executable instructions which are executable by a processor for performing any of the aforementioned management operations. The databases 704 - 716 may be any structure and/or structures suitable for storing the records 728 - 738 and/or the rules 740 - 752 (e.g., an active database, a relational database, a table, a matrix, an array, a flat file, and the like).

The signal records 728 may include a variety of signals, along with associated metadata. For example, the signals may comprise optical signals (e.g., single-channel and/or multi-channel PPG signals) and/or non-optical signals (e.g., pressure pulse signals). In some embodiments, the metadata may include information obtained from the signals, such as heart rate(s) of an associated user. For example, the signal records 728 may store some or all of the following information:

- Signal(s) Identifier: Identifier that identifies the stored signal(s).

- Signal(s): one or more signals. The signals may be raw signals (e.g., as detected by the associated blood metrics measurement apparatus ), filtered or pre-processed signals (e.g., to remove noise from the signals), and/or normalized signal values (e.g., between 0 - 1). It will be appreciated that as used in this paper, a "signal," such as a PPG signal or pressure pulse signal, generally refers to a filtered signal, although in some embodiments, it may also refer to an unfiltered signal instead of, or in addition to, the filtered signal.

- Set(s) of Waves: one or more sets of waves of a predetermined time series (e.g., 8 seconds) of the signal(s). The set of waves may include raw waves, filtered waves, and/or normalized wave values (e.g., between 0 - 1).

- Apparatus Identifier: Identifier that identifies the blood metrics measurement apparatus that generated the signals.

- User Account Identifier: Identifier that identifies a user account associated with the blood metrics measurement apparatus that generated the signals.

- Metadata: Metadata obtained from the signals, such as heart rate or other biometric data. The metadata may also include other information of the user, such as gender, age, height, weight, skin color (e.g., obtained from the user's account information). Such metadata values may be used by the blood pressure calculation module 724 (discussed below) to facilitate calculation of arterial blood pressure values. In some embodiments, metadata values may be provided to an empirical blood pressure model (discussed below) via sets of feature vectors (discussed below) and/or be provided separately to the model.

The wave records 730 may include sets of waves of a signal (e.g., a signal stored in the signal database 704), along with subsets of those waves. The subsets of waves may comprise "high quality" waves obtained from the waves of the signal. These subsets of waves may provide, for example, a more accurate blood pressure calculation that just using the signal or waves of the signal. In some embodiments, the wave records 730 may store some or all of the following information:

- Signal Identifier: Identifier that identifies an associated signal.

- Wave Identifier(s): Identifiers for subsets of waves of the associated signal.

- Subset(s) of Waves: one or more subsets of waves of the associated signal. The subsets of waves may include raw waves, filtered waves, and/or normalized wave values (e.g., between 0 - 1). The subsets of waves may be referred to as "high quality" waves.

- Apparatus Identifier: Identifier that identifies the blood metrics measurement apparatus that generated the signals.

- User Account Identifier: Identifier that identifies a user account associated with the blood metrics measurement apparatus that generated the signals.

The wave feature records 732 may include wave features of associated subsets of waves of a signal. For example, wave features may include wave peaks, wave valleys, wave edges, and/or the like. In some embodiments, the wave feature records 732 may store some or all of the following information:

- Signal Identifier: Identifier that identifies an associated signal.

- Wave Identifier(s): Identifiers for the subsets of waves of the associated signal.

- Wave Features: one or more features obtained from the waves within the associated subsets of waves. The wave features may include points of the waves, such as wave peaks, wave valleys, wave edges, and/or the like. The wave features may be stored as normalized wave values (e.g., between 0 - 1).

- Apparatus Identifier: Identifier that identifies the blood metrics measurement apparatus that generated the signals.

- User Account Identifier: Identifier that identifies a user account associated with the blood metrics measurement apparatus that generated the signals.

The feature vector records 734 may include sets of features generated based on the wave features of associated subsets of waves of a signal. In some embodiments, the feature vector records 734 may store some or all of the following information:

- Signal Identifier: Identifier that identifies an associated signal.

- Wave Identifier(s): Identifiers for the subsets of waves of the associated signal.

- Set(s) of Feature Vectors: one or more sets of feature vectors, each feature vector comprising features extracted from a wave of an associated subset of waves. The values of a feature vector may include measurement values and metric values. For example, the measurement values may correspond to amplitude or location points of a particular wave, and the metric values may be generated from metric functions that use at least one of the measurement values. The values of a feature vector may comprise normalized values (e.g., between 0 - 1).

- Apparatus Identifier: Identifier that identifies the blood metrics measurement apparatus that generated the signals.

- User Account Identifier: Identifier that identifies a user account associated with the blood metrics measurement apparatus that generated the signals.

The blood pressure model records 736 may include one or more empirical blood pressure models. The models may include various types of empirical blood pressure models. For example, a first type may be a "non-specific" model which does not require calibration in order to be used to calculate arterial blood pressure values. A second type may be a "specific" model which requires calibration in order to be used to calculate arterial blood pressure values. For example, models of the second type may require information about the user, such age, weight, height, gender, skin color, and/or the like. In some embodiments, the blood pressure records 736 may store some or all of the following information:

- Model Identifier: Identifies an empirical blood pressure model.

- Model Type: Identifies a type of model (e.g., non-specific or specific).

- Model Parameters: Various model parameters (e.g., decision node parameters) and tree structures used to calculate the arterial blood pressure values based on the sets of feature vectors and/or other related information (e.g., gender, age, weight, height, skin color, etc.).

- Apparatus Identifier(s): Identifier(s) that identify one or more blood metrics measurement apparatus' using the empirical blood pressure model.

- User Account Identifier(s): Identifier(s) that identify one or more user account(s) associated with the blood metrics measurement apparatus that use the empirical blood pressure model.

The blood pressure results records 738 may include one or more calculated arterial blood pressure values. In some embodiments, the blood pressure results records 738 may store some or all of the following information:

- Blood Pressure Result Identifier: Identifies a set of one or more calculated arterial blood pressure values.

- Blood Pressure Values: one or more calculated arterial blood pressure values.

- Date: A date and/or time the arterial blood pressure was calculated.

- Messages: Message identifier and/or messages generated based on the calculated blood pressure values.

- Signal Identifier: Identifier that identifies an associated signal.

- Wave Identifier(s): Identifiers for the subsets of waves of the associated signal.

- Feature Vectors Identifier(s): Identifiers for the one or more sets of feature vectors used to calculate the arterial blood pressure values.

- Apparatus Identifier: Identifier that identifies the blood metrics measurement apparatus that generated the signals.

- User Account Identifier: Identifier that identifies a user account associated with the blood metrics measurement apparatus that generated the signals.

*Pre-Processing Rules 740*

The pre-processing rules 740 define attributes and/or functions for filtering signals. For example, a signal may be corrupted with noise from various sources (e.g., high frequency ambient noise, electronic noise, and the like). In some embodiments, the signal may be the raw signal data from a photodiode, output of a pressure sensor, output of an accelerometer, output of a gyroscope, and the like. The signal may be filtered to remove corrupting noise, as well as enhance a strength of the signal.

The pre-processing module 718 may be configured to execute the pre-processing rules 740. Thus, for example, the pre-processing module 718, using some or all of the associated signals and/or values stored in the signal records 728, may filter signals and store the resulting filtered signals. The wave database 706 may be configured to store the signals filtered by the pre-processing module 718.

*Wave Selection Rules 742*

The wave selection rules 742 define attributes and/or functions for selecting (or, "extracting") high quality waves from a set of waves of a signal. The high quality waves may form a subset of waves.

In some embodiments, signal measurements are sensitive to motion, pressure, and ambient light distortions. Noise-removal filters may not be entirely effective when there is intense noise. It may be helpful to select high quality signal waves from measured time-series data to reduce or eliminate the effects of motion, pressure, and/or ambient light distortions.

In some embodiments, the wave selection rules 742 identifies candidate waves from a signal, and determines whether any of the candidate waves satisfy a model match criterion (e.g., a Gaussian mixture model or group similarity model). Candidate waves that pass the model match criterion may be classified as high quality waves. In some embodiments, candidate waves includes waves having valleys whose frequency match, or substantially match, the heart rate of the user or other heart rate value (e.g. a default heart rate value).

A group similarity model may be preferable. For example, while a Gaussian mixture model may perform well with "smoothed" signals (e.g., low pass filtered at 4-6 Hz), the Gaussian mixture model may fail, or perform poorly, if the signal includes more features. In some embodiments, the Gaussian mixture model may be unable to match more than 2 peak results in a reflection of waves that reveal features critical in the prediction (or, "estimation) of blood pressure. In some embodiments, additional Gaussian mixture models may be incorporated to address such issues, although this may lead to added complexity in optimization (e.g., model fitting), as well as added constraints to ensure specificity to physiologically meaningful signals against noise.

In some embodiments, a group similarity model may overcome some or all of the limitations described above. Generally, high quality (or high fidelity) waves that correspond with arterial blood flow corresponding to a single cycle typically occur in groups. In cases where there is motion, it may be unlikely that an individual wave would have a high quality when waves before it were not of high quality. In the group similarity model, candidate waves may be individually matched to an existing wave buffer (e.g., of the wave database 706). In some embodiments, the wave buffer may include some or all previous candidates waves added in succession in a first in first out (FIFO) method. The size of the wave buffer may be set to include a predetermined number of waves (e.g., 15 waves), and may be altered. For example, increasing the wave buffer to include additional candidate waves may require that candidate waves match a larger set of previously extracted candidate waves. In some embodiments, in order to measure similarity, the candidate waves may be re-parameterized to a fixed length (e.g., using a cubic spline).

In various embodiments, the group similarity model applies a similarity measure to determine if, and to what extent, a candidate wave matches some or all of the existing waves in the wave buffer. In one example, the similarity measure may be an average correlation between the candidate wave and the individual waves in the wave buffer. In other embodiments, the similarity measure may include a like sum of squared differences, joint entropy or mutual information, and the like. In this example, if the similarity measure is greater than a threshold (e.g. 0.95 for correlation), this wave is selected for further processing. In some embodiments, both the candidate waves that are selected for further processing and the candidate waves that are not selected for further processing may be added to the wave buffer (e.g., for subsequent applications of the group similarity model).

The group similarity model may process waves with multiple reflections. In some embodiments, the group similarity model may continuously update a template (e.g., the wave buffer) against which incoming candidate waves are matched, and that template may indicate the true underlying signal, which may be different from user to user, instead of relying on a predetermined template. The group similarity model may reduce false positive identification of candidate waves relative to other models (e.g., Gaussian mixture model).

The wave selection module 720 may be configured to execute the wave selection rules 742. Thus, for example, the wave selection module 720, using some or all of the associated waves and/or values stored in the signal records 728, may identify one or more subsets of "high quality" waves. The wave database 706 may be configured to store the subsets of waves identified by the wave selection module 720.

*Feature Extraction Rules 744*

The feature extraction rules 744 define attributes and/or functions for identifying (or, "extracting") features from waves (e.g., high quality waves). In some embodiments, features may be concatenated to form feature vectors.

Features may include, but are not limited to, pulse transit time (PTT) features, reflection features, signal level and range features, signal metric features, optical ratio features, heart rate features, wave width and derivative features, user information features, and/or pressure features. In some embodiments, PTT features include a transit time feature, modified pulse transit time feature, joint entropy feature, and wave type feature. Pulse transit time may be measured as the time taken for the pulse pressure wave to propagate along the length of the arterial tree. For example, this may be the difference in time between the onset of the R-wave on ECG, and the pulse wave peak on the finger. In other embodiments, the transit time may be measured as the time taken for blood to travel from one LED-PD system to another LED-PD system (e.g., as depicted in FIG. 3B), which may be separated from each other by a predetermined distance (e.g., 15 mm). Sampling at a very high rate (>2 KHz) may help resolve features in both LED-PD systems. In some embodiments, the transit time may be measured as (1) the distance between the valleys in the corresponding waves in respective LED-PD systems at the start of the systolic cycle, and/or (2) the distance between the peaks in first derivatives between the two LED-PD systems. The transit time may be expressed in any units (e.g., milliseconds).

The joint entropy feature may be a measurement of similarity between fast channels of respective LED-PD systems. High pulse pressure may correlate with low entropy or high mutual information shared between the fast LED channels. The joint entropy feature may be expressed in bytes.

Wave type features may include different types of waves. For example, different types of waves may include slow type waves and fast type waves. In some embodiments, the wave types feature quantifies the relative position of the systolic peak within the wave (0<WaveType<1) for each of the fast channels in respective LED-PD systems, where small values correspond to a slow type waves and larger values correspond to fast type waves.

A pulse decomposition may track a pulse pressure wave. As the pressure wave travels in the arterial systems, it may encounter branching points where the diameter of arteries may decrease rapidly. The pressure wave may bounce on such branching points and send reflection waves in different (e.g., opposite) directions. In some embodiments, the original pressure wave plus the reflections may form the observed pressure pulse, which in turn may be observed in a signal (e.g., PPG signal). Multiple reflections (e.g., four reflections) may be used to identify multiple features and amplitudes (e.g., four features and four amplitudes). The time of occurrence of the systolic peak from the start of the systolic cycle (i.e., systolic peak time) may also be included as a feature. In some embodiments, a ratio between the second derivatives at the bottom and peak of the systolic cycle may be used as a feature. This feature may be correlated with arterial stiffness. In some embodiments, ten features may be individually identified for each LED channel.

The signal level and range features may comprise the mean value and range of a signal (e.g., PPG signal) determined for each of the channels. In some embodiments, the signal metrics features include Hjorth parameters, perfusion, kurtosis, and energy features. Hjorth parameters may describe activity, mobility and complexity of the signal, and are commonly used tools in EEG analysis. Perfusion may be measured as the ratio between the AC and DC components of the signal (i.e. the range of the signal and the mean). Kurtosis may be measured over the signal analysis window. Energy features may include variance of the signal measured over the analysis window. In some embodiments, optical ratio features may be calculated as the ratio of the range of the LEDs (measured in pairs across wavelengths) after normalization of the respective signals, and heart rate features may be an actual user heart rate or a default heart rate.

In some embodiments, the wave width and derivative features may include time distances within a wave to its main peak at different amplitude locations. The secondary (diastolic) peak may be extracted from the first order derivative (FOD) of the wave. For this, FOD may be smoothed (e.g., a simple moving average filter may be used). In some embodiments, after extracting main (systolic) and secondary (diastolic) peaks, reflection (augmentation) index (the ratio of diastolic peak and systolic peak amplitudes), inflection point area ratio (the ratio of areas under the wave that are separated by the diastolic inflection point), and/or stiffness index (the ratio of patient's height to the time distance between the systolic and diastolic peaks) may be determined from the first order derivative of the wave.

In one example, the second order derivative of the wave has multiple peaks and valley points (e.g., labeled (a), (b) and (e)). In some embodiments additional peak and valleys may be present. If the sampling frequency is low or under exercise conditions, only waves (a), (b) and (e) may be identifiable. In some embodiments, for higher sampling frequencies (e.g., ≥200Hz), additional peaks and valleys may be identifiable. The ratio of these values may be included into a feature vector.

In some embodiments, if two LEDs at the same wavelength are positioned at different locations of the same artery, a phase shift may be obtained between the measured signals (e.g., due to blood flow). This may facilitate calculation of pulse wave velocity (PWV) and/or pulse transit time (PTT), both of which may be included in a feature vector, and/or otherwise provided to the empirical blood pressure model used to calculate arterial blood pressure values.

The user information features may include, for example, a user's gender, age, skin color, height and/or weight. The user information may be included in a feature vector, and/or otherwise provided to the empirical blood pressure model used to calculate arterial blood pressure values. In some embodiments, pressure features may include the mean value of the pressure signal over the analysis window (or "baseline mean") and range of the pressure signal over the analysis window (or, "AC").

The feature extraction module 722 may be configured to execute the feature extraction rules 744. Thus, for example, the feature extraction module 720, using some or all of the associated subsets of waves and/or values stored in the wave records 730, may identify one or more feature of the waves within the subsets of waves, and generate corresponding sets of feature vectors. The wave feature database 708 may be configured to store the wave features identified by the feature extraction module 722, and the feature vector database 710 may be configured to store the generated sets of feature vectors.

*Blood Pressure Processing Rules 746*

The blood pressure processing rules 746 define attributes and/or functions for calculating arterial blood pressure values of a user. In some embodiments, the blood pressure processing rules 746 specify, identify, and/or define the empirical blood pressure model to use for calculating arterial blood pressure of a user. The rules 746 may further define input values for the empirical blood pressure model. For example, input values may comprises the sets of features vectors, and/or other attributes of the user (e.g., age, gender, height, weight, skin color, etc.), assuming such attributes have not been included in the feature vectors.

The blood pressure processing module 724 may be configured to execute the blood pressure processing rules 746, the channel selection rules, and the modified pulse transit time rules. Thus, for example, the blood pressure processing module 724 may select a channel for calculating modified pulse transit time, calculate modified pulse transit time, and calculate a blood pressure based on modified pulse transit time.

In some embodiments, the blood pressure processing module 724, using an empirical blood pressure model stored in the blood pressure model database 712, along with some or all of the associated sets of feature vectors and/or values stored in the feature vector records 738, may calculate one or more arterial blood pressure values. The blood pressure results database 714 may be configured to store the blood pressure values calculated by the blood pressure processing module 724.

*Message Rules 748*

The message rules 748 define attributes and/or functions for generating messages and/or alerts based on arterial blood pressure values. In some embodiments, the message rules 748 may define rules that cause the blood pressure calculation system 314 to provide calculate blood pressure values to a user. In some embodiments, the message rules 748 may include threshold values and/or conditions that when exceeded and/or satisfied, trigger a message or alert. For example, a threshold value (or value range) and/or threshold condition may be associated with varying blood pressure levels (e.g., hypotension, normal blood pressure, prehypertension, stage 1 hypertension, stage 2 hypertension, etc.), and a calculated blood pressure value which satisfies a corresponding threshold condition or value may trigger a message or alert (e.g., indicating the corresponding blood pressure level).

In some embodiments, the communication module 726 may be configured to execute the message rules 748. Thus, for example, the communication module 720, using some or all of the blood pressure values stored in the blood pressure results records 738, may generate one or more messages. The communication module 726 may be configured to provide those messages to a user.

In some embodiments, the communication module 726 may be configured to send requests to and receive data from one or a plurality of systems. The communication module 726 may send requests to and receive data from a systems through a network or a portion of a network. Depending upon implementation-specific or other considerations, the communication module 726 may send requests and receive data through a connection, and/or the communication link 910), all or a portion of which may be a wireless connection. The communication module 726 may request and receive messages, and/or other communications from associated systems.

*Channel Selection Rules 750*

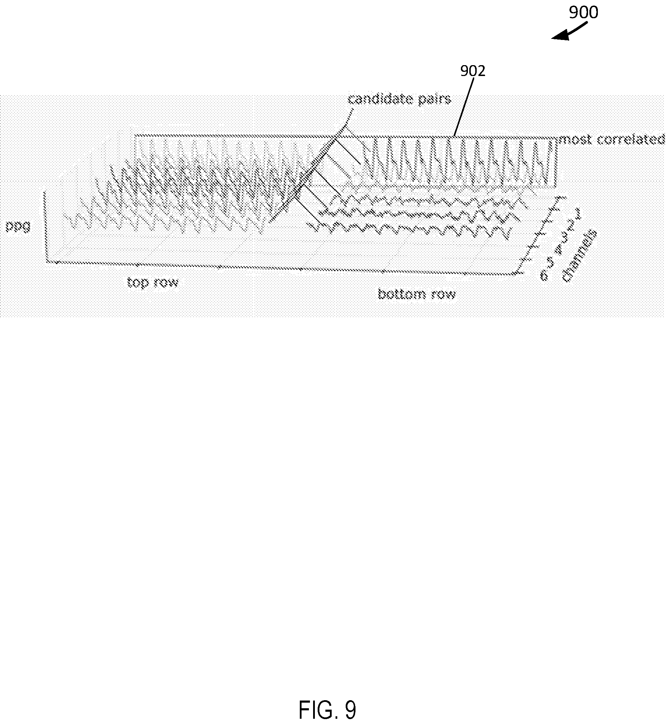

The channel selection rules 748 define attributes and/or functions for selecting one or more channels for determining modified pulse transit time. Generally, in order to maintain good signal quality, an array of sensors may be used which facilitates sampling of PPG signals at any number of locations (e.g., 12 different locations). In some embodiments, to compute modified pulse transit time, two channels with good arterial signal may be selected. In one example, this may be performed by selecting the two channels amongst a list of candidate pairs that have the highest correlation. High positive correlation between two channels may indicate the SNR is high, and that they both correspond to arterial signal; when placed on top of the artery, a channel outputs a signal with much larger amplitude than when on top of a vein, which results in a lower SNR. A negative correlation may indicate that one of the channels is more venous, since the venous blood flows backward with a small delay. The candidate pair may be chosen by estimating a possible path for the artery as it crosses the measurement apparatus. FIG. 9 depicts a graph 900 of example signal channels including an example pair of signal channels 902 selected for calculation of modified pulse transit time. In various embodiments, the channel selection rules 750 are defined and/or configured for execution by the blood metrics measurement apparatus 200.

In some embodiments, the channel selection rules 750 may define some or all of the following steps:

1. Gather 4 to 8 seconds of data from each of the channels (e.g., 12 channels).

2. Apply a simple high pass filter (cutoff ∼ 0.7 Hz) to remove large motion artifacts (optional).

3. Compute Pearson correlation coefficient:

$$c = <(S1\text{-}s1avg)/\|S1\|, (S2\text{-}s2av)/\|S2\|>$$

between each of the pair candidates time series S1 and S2 (with <,> the dot product and ||.|| the L2 norm).

4. Select channel pair that gives the highest correlation for the calculation of modified pulse transit time.

In some embodiments, once a pair of channels is selected, data from each channel is input into the modified pulse transit time method described below. As noted above, FIG. 9 depicts a graph 900 of example signal channels including an example pair of signal channels 902 selected for calculation of modified pulse transit time.

*Modified Pulse Transit Time Rules 752*

The modified pulse transit time rules 752 define attributes and/or functions for determining modified pulse transit time.

Phase Pulse Transit (Frequency Domain)

Phase methods compute pulse transit time in the frequency domain where the phase difference between two different signals is a continuous value, so it may achieve subsample resolution without oversampling the signal. Phase difference, or time delay, can be computed between two signals by using a frequency domain transform function (e.g., Fourier transform) of each signal, and calling a 4-quadrant inverse tangent function on the imaginary and real parts of each frequency component. This can provide a phase value for each frequency component for each signal, and taking a difference for each frequency phase value may yield a phase difference which may be converted to a time value (e.g., time shift between the signals).

Heart Rate Phase

Heart rate phase is an evolution of the phase method which, in some embodiments, only considers frequency components close to the estimated heart rate, which may reduce the potential for noisy frequency components in the computation.

An example flow of computation may include some or all of the following:

1. Compute Fourier transforms s[1_S]^and sQ2_S]^of input signals s1 and s2

2. Find an estimate of the heart rate as the frequency that corresponds to the largest amplitude of the combined spectrum, calculated as the sum of the squared amplitude of the two transformed signals:

$$HR[\![\_est]\!]=max\_freq\ (s[\![1\_S]\!]^2+s[\![2\_s]\!]^2)$$

3. Compute component-wise phases of the two signals

$$\varphi\_1=tan^{(-1)}\ (Im(s1\_s)/Real(s1\_s)), \varphi\_2=tan^{(-1)}\ (Im(s2\_s)/Real(s2\_s))$$

4. Compute phase differences for frequency components at or near the heart rate and any desired number of harmonic frequencies which also contribute to the heart rate signal (typically 0-20 harmonics).

5. Filter and combine all individually computed phase differences. Filtering here corresponds to discarding unrealistic values, i.e. outside the expected physiological range, and taking the averages of the values within a frequency band that would corresponds to the different features of the arterial pressure wave, such as 0.8 to 20 Hz.

Shift-Methods (Time Domain)

*Linear shift*

Shift methods corresponds to a group of methods which use the shifting of signals relative to one another until a metric is minimized or maximized. The time or sample shift which produces the best metric value is the pulse transit time.

Cost Functions:

1. Joint entropy. The Shannon entropy of the two signals, minimized, or a related measure called mutual information between the signals, maximized.

2. Correlation coefficient. The Pearson Correlation coefficient, maximized.

In some embodiments, a cost function is computed at each relative shift of two signals to determine the modified pulse transit time. This shifting may be performed by delaying one signal by a specific number of samples, and then creating a window of points over a time period in which both signals have valid samples. The window gives two equal length signal segments on which the cost function is computed.

In one example, the signals are delayed or shifted by an integer number of samples, but to resolve finer details signals can be interpolated and resampled in the time domain (linear, sinc, spline, or other interpolation methods), or shifted in the frequency domain such that subsample shifts are possible. In some embodiments, the correlation coefficient cost and single sample shifting are referred to as cross-correlation.

*Non-Linear Shift*

In one example, a correlation time of the pulse transit time value may be less than the width of the window used for calculation, and constant shift methods may not be effective. To overcome this issue, Dynamic Time Warping (or, "DTW"), which may include identifying the best non-linear shift between two time series to minimize some cost function representing the alignment between the two series. Such a cost function can be the L1 norm between the two signals, or other distance measure.

In some embodiments, dynamic time warping calculates the "distance" between each possible pair of points belonging to either signal, then progresses along the cost matrix that stores all those distance in such a manner to minimize the cumulative cost between the two signals.

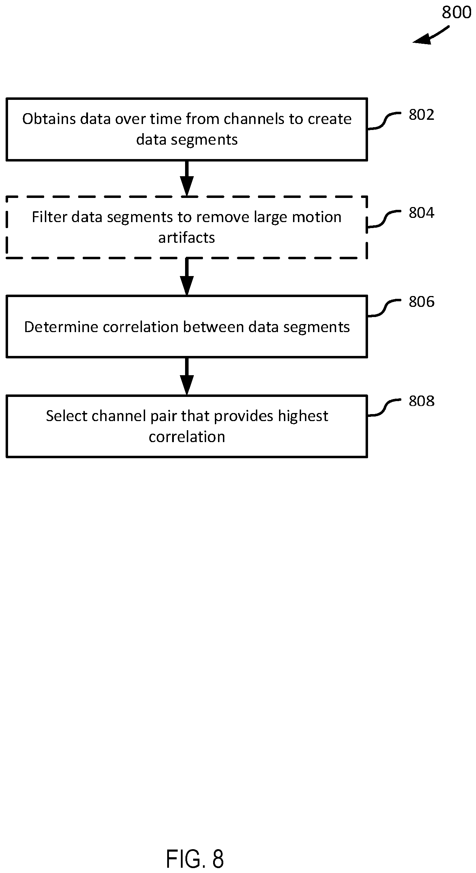

FIG. 8 depicts a flowchart 800 of an example method of channel selection according to some embodiments.

In step 802, a wearable blood metrics measurement apparatus (e.g., blood metrics measurement apparatus 200) obtains data over time from signal channels to create data segments. In some embodiments, a blood pressure processing module (e.g., blood pressure processing module 724) of a blood pressure calculation system (e.g., blood calculation system 314) uses one or more channel selection rules (e.g., channel selection rules 750) to obtain the data.

In step 804, the wearable blood metrics measurement apparatus filters the data segments to remove large motion artifacts. In some embodiments, the blood pressure processing module of the blood pressure calculation system uses one or more channel selection rules to filter the data segments.

In step 806, the wearable blood metrics measurement apparatus determines correlation between data segments. In some embodiments, the blood pressure processing module of the blood pressure calculation system uses one or more channel selection rules to perform the determination.

In step 808, the wearable blood metrics measurement apparatus selects the channel pair that provides highest correlation. In some embodiments, the blood pressure processing module of the blood pressure calculation system uses one or more channel selection rules to perform the selection.

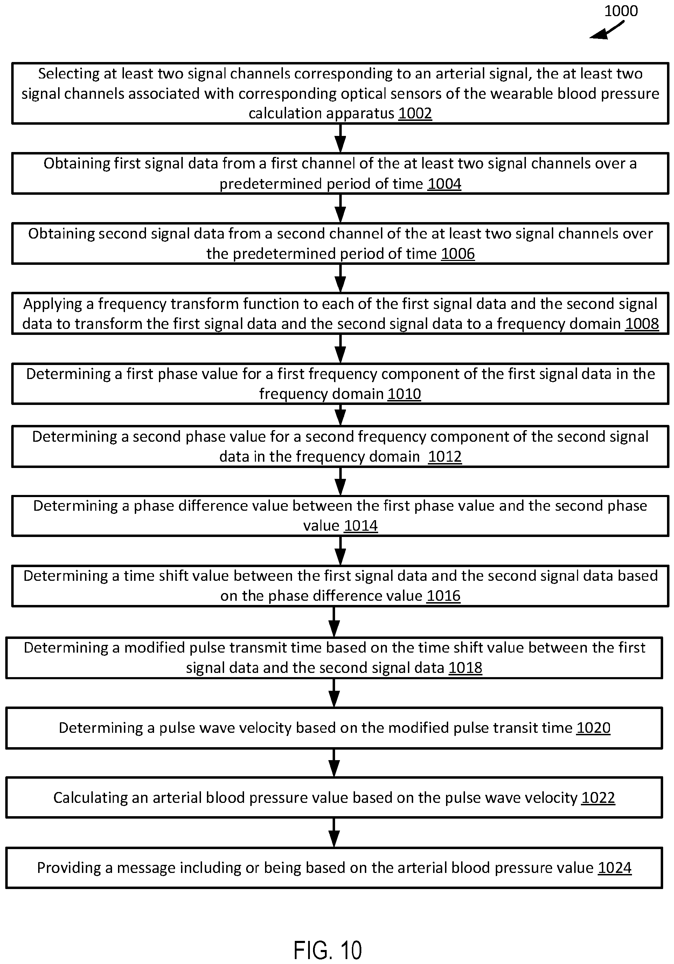

FIG. 10 depicts a flowchart 1000 of an example method of calculating blood pressure according to some embodiments.

In step 1002, a wearable blood metrics measurement apparatus (e.g., blood metrics measurement apparatus 200) selects at least two signal channels corresponding to an arterial signal, the at least two signal channels associated with corresponding optical sensors of the wearable blood metrics measurement apparatus. In some embodiments, a blood pressure processing module (e.g., blood pressure processing module 724) of a blood pressure calculation system (e.g., blood calculation system 314) uses one or more channel selection rules (e.g., channel selection rules 750) to perform the selection.

In step 1004, the wearable blood metrics measurement apparatus obtains first signal data (e.g., PPG signal data) from a first channel of the at least two signal channels (e.g., a pair of signal channels) over a predetermined period of time. In some embodiments, the blood pressure processing module of the blood pressure calculation systems uses one or more modified pulse transit time rules (e.g., modified pulse transit time rules 752) to obtain the first signal data.

In step 1006, the wearable blood metrics measurement apparatus obtains second signal data from a second channel of the at least two signal channels over the predetermined period of time. In some embodiments, the blood pressure processing module of the blood pressure calculation systems uses one or more modified pulse transit time rules to obtain the first signal data to obtain the second signal data.

In step 1008, the wearable blood metrics measurement apparatus applies a frequency transform function (e.g., a Fourier transform) to each of the first signal data and the second signal data to a transform the first signal data and the second signal data to a frequency domain. In some embodiments, the blood pressure processing module of the blood pressure calculation systems uses one or more modified pulse transit time rules to apply the frequency transform function.

In step 1010, the wearable blood metrics measurement apparatus determines a first phase value for a first frequency component of the first signal data in the frequency domain. In some embodiments, the blood pressure processing module of the blood pressure calculation systems uses one or more modified pulse transit time rules to perform the determination.

In step 1012, the wearable blood metrics measurement apparatus determines a second phase value for a second frequency component of the second signal data in the frequency domain

In step 1014, the wearable blood metrics measurement apparatus determines a phase difference value between the first phase value and the second phase value. In some embodiments, the blood pressure processing module of the blood pressure calculation systems uses one or more modified pulse transit time rules to perform the determination.

In step 1016, the wearable blood metrics measurement apparatus determines a time shift value between the first signal data and the second signal data based on the phase difference value. In some embodiments, the blood pressure processing module of the blood pressure calculation systems uses one or more modified pulse transit time rules to perform the determination.

In step 1018, the wearable blood metrics measurement apparatus determines a modified pulse transmit time based on the time shift value between the first signal data and the second signal data, the modified pulse transit time representing a transit time for a pressure wavefront to travel from a first optical sensor of the corresponding optical sensors and a second optical sensor of the corresponding optical sensors. In some embodiments, the blood pressure processing module of the blood pressure calculation systems uses one or more modified pulse transit time rules to perform the determination.

In step 1020, the wearable blood metrics measurement apparatus determines a pulse wave velocity based on the modified pulse transit time. In some embodiments, the blood pressure processing module of the blood pressure calculation systems uses one or more modified pulse transit time rules to perform the determination.

In step 1022, the wearable blood metrics measurement apparatus calculates an arterial blood pressure value based on the pulse wave velocity. In some embodiments, the blood pressure processing module of the blood pressure calculation systems uses one or more modified pulse transit time rules and/or one or more blood pressure processing rules (e.g., blood pressure processing rules 746) to perform the calculation.

In step 1024, the wearable blood metrics measurement apparatus provides a message including or being based on the arterial blood pressure value. In some embodiments, a user interface module (e.g., user interface module 316) of the blood metrics measurement apparatus provides the message using one or more message rules (e.g., message rules 748).

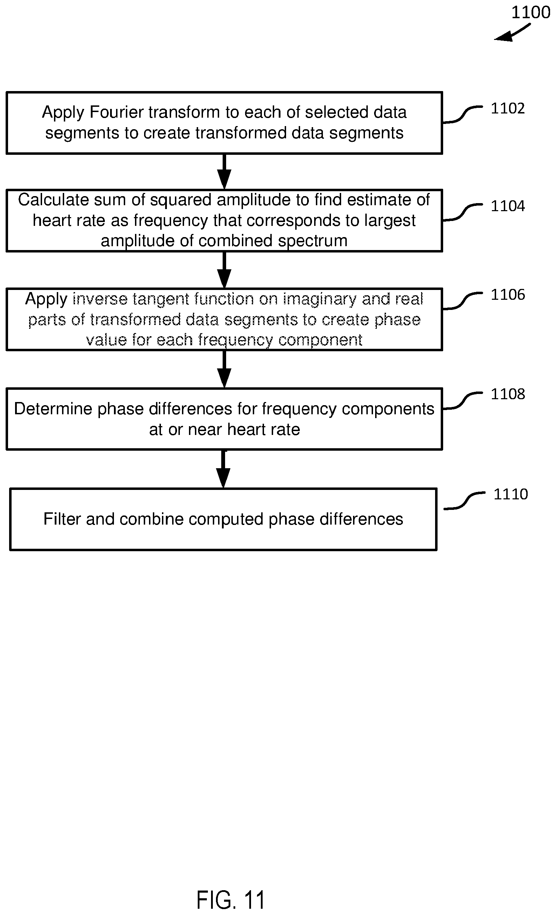

FIG. 11 depicts a flowchart 1100 of an example heart rate phase method according to some embodiments.

In step 1102, a wearable blood metrics measurement apparatus (e.g., blood metrics measurement apparatus 200) applies a frequency domain transform function (e.g., Fourier transform) to each of selected data segments to create transformed data segments. In some embodiments, a blood pressure processing module (e.g., blood pressure processing module 724) of a blood pressure calculation system (e.g., blood calculation system 314) uses one or more modified pulse transit time rules (e.g., modified pulse transit time rules 752) to apply the transform.

In step 1104, the wearable blood metrics measurement apparatus calculates a sum of squared amplitude to find an estimate of heart rate as a frequency that corresponds to the largest amplitude of combined spectrum. In some embodiments, the blood pressure processing module of the blood pressure calculation systems uses one or more modified pulse transit time rules to perform the calculation.

In step 1106, the wearable blood metrics measurement apparatus applies an inverse tangent function on imaginary and real parts of transformed data segments to create phase values for each frequency component. In some embodiments, the blood pressure processing module of the blood pressure calculation systems uses one or more modified pulse transit time rules to create the phase values.

In step 1108, the wearable blood metrics measurement apparatus determines a phase differences for frequency components at or near heart rate. In some embodiments, the blood pressure processing module of the blood pressure calculation systems uses one or more modified pulse transit time rules to perform the determination.

In step 1110, the wearable blood metrics measurement apparatus filters and combines computed phase differences. In some embodiments, the blood pressure processing module of the blood pressure calculation systems uses one or more modified pulse transit time rules to filter and combine the computer phase differences.

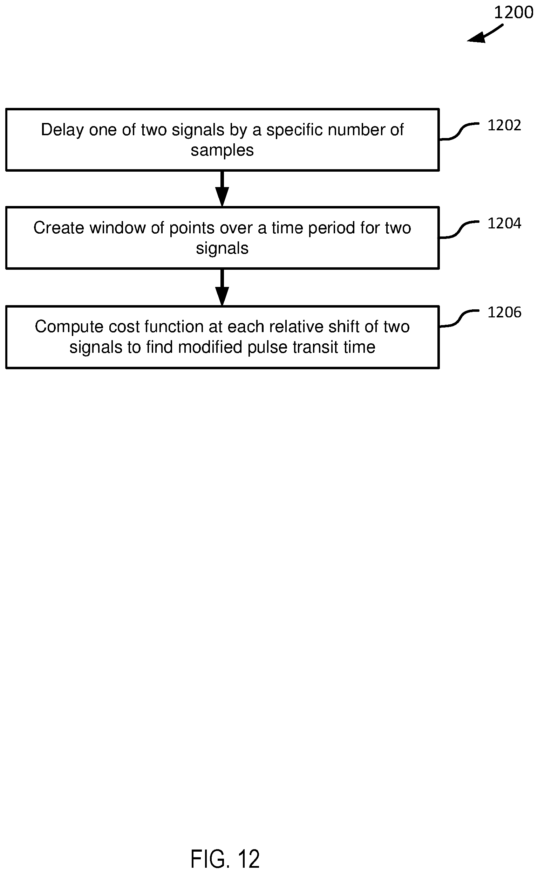

FIG. 12 depicts a flowchart 1200 of an example linear shift method according to some embodiments.

In step 1202, a wearable blood metrics measurement apparatus (e.g., blood metrics measurement apparatus 200) delays one of two signals by a specific number of samples. In some embodiments, a blood pressure processing module (e.g., blood pressure processing module 724) of a blood pressure calculation system (e.g., blood calculation system 314) uses one or more modified pulse transit time rules (e.g., modified pulse transit time rules 752) to apply the transform.

In step 1204, the wearable blood metrics measurement apparatus creates a window of points over a time for two signals. In some embodiments, the blood pressure processing module of the blood pressure calculation systems uses one or more modified pulse transit time rules to create the window of points over the time for two signals.

In step 1206, the wearable blood metrics measurement apparatus computes a cost function at each relative shift of two signals to find the modified pulse transit time. In some embodiments, the blood pressure processing module of the blood pressure calculation systems uses one or more modified pulse transit time rules to compute the cost function and find the modified pulse transit time.

FIG. 13 is a block diagram of an example digital device 1300. The digital device 1300 comprises a processor 1302, a memory system 1304, a storage system 1306, a communication network interface 1308, an I/O interface 1310, and a display interface 1312 communicatively coupled to a bus 1314. The processor 1302 is configured to execute executable instructions (e.g., programs). In some embodiments, the processor 1302 comprises circuitry or any processor capable of processing the executable instructions.

The memory system 1304 is any memory configured to store data. Some examples of the memory system 1304 are storage devices, such as RAM or ROM. The memory system 1304 can comprise the RAM cache. In various embodiments, data is stored within the memory system 1304. The data within the memory system 1304 may be cleared or ultimately transferred to the storage system 1306.

The storage system 1306 is any storage configured to retrieve and store data. Some examples of the storage system 1306 are flash drives, hard drives, optical drives, and/or magnetic tape. In some embodiments, the digital device 1300 includes a memory system 1304 in the form of RAM and a storage system 1306 in the form of flash data. Both the memory system 1304 and the storage system 1306 comprise computer readable media which may store instructions or programs that are executable by a computer processor including the processor 1302.

The communications network interface (com. network interface) 1308 can be coupled to a network via the link 1316. The communication network interface 1308 may support communication over an Ethernet connection, a serial connection, a parallel connection, or an ATA connection, for example. The communication network interface 1308 may also support wireless communication (e.g., 802.11 a/b/g/n, WiMax). It will be appreciated that the communication network interface 1308 can support many wired and wireless standards.

The optional input/output (I/O) interface 1310 is any device that receives input from the user and output data. The optional display interface 1312 is any device that is configured to output graphics and data to a display. In one example, the display interface 1312 is a graphics adapter.

It will be appreciated that the hardware elements of the digital device 1300 are not limited to those depicted in FIG. 13. A digital device 1300 may comprise more or less hardware elements than those depicted. Further, hardware elements may share functionality and still be within various embodiments described herein. In one example, encoding and/or decoding may be performed by the processor 1302 and/or a co-processor located on a GPU (e.g., NVidia®).

It will be appreciated that a "user device," "apparatus," "server," "module," "system," and/or "database" may comprise software, hardware, firmware, and/or circuitry. In one example, one or more software programs comprising instructions capable of being executable by one or more processors (e.g., hardware processors) may perform one or more of the functions of the user devices, servers, modules, systems, and/or databases described herein. In another example, circuitry may perform the same or similar functions. Alternative embodiments may comprise more, less, or functionally equivalent user devices, apparatus, servers, modules, systems, and/or databases, and still be within the scope of present embodiments. For example, some or all features of the blood metrics measurement apparatus 200 may be performed by an associated user device (e.g., mobile device, smartphone, tablet, laptop computer, desktop computer, tablet computer, and the like).

The invention is defined by the appended claims.

## Claims

1. A wearable blood metrics measurement apparatus (200) comprising:

one or more processors (1302);
memory (1304) storing instructions that, when executed by the one or more processors (1302), cause the wearable blood metrics measurement apparatus to perform:

selecting (1002) at least two signal channels corresponding to an arterial signal, the at least two signal channels associated with corresponding optical sensors of the wearable blood metrics measurement apparatus spatially separated by a predetermined distance;
obtaining (1004) first signal data from a first channel of the at least two signal channels over a predetermined period of time;
obtaining (1006) second signal data from a second channel of the at least two signal channels over the predetermined period of time;
determining a difference between the first signal data and a predetermined value;
determining a difference between the second signal data and the predetermined value;
if the difference between the first signal data and the predetermined value is greater than a threshold, ignoring that first signal data;
if the difference between the second signal data and the predetermined value is greater than the threshold, ignoring the second signal data;
applying (1008) a frequency transform function to each of the first signal data and the second signal data to transform the first signal data and the second signal data to a frequency domain; determining (1010) a first phase value for a first frequency component of the first signal data in the frequency domain;
determining (1012) a second phase value for a second frequency component of the second signal data in the frequency domain;
determining (1014) a phase difference value between the first phase value and the second phase value;
determining (1016) a time shift value between the first signal data and the second signal data based on the phase difference value;
determining a modified pulse transit time (1018) based on the time shift value between the first signal data and the second signal data, the modified pulse transit time representing a transit time for a pressure wavefront to travel from a first optical sensor of the corresponding optical sensors to a second optical sensor of the corresponding optical sensors;
determining a pulse wave velocity (1020) based on the predetermined distance over the modified pulse transit time;
calculating an arterial blood pressure value (1022) based on the pulse wave velocity; and
providing (1024) a message including or being based on the arterial blood pressure value.

2. The wearable blood metrics measurement apparatus (200) of claim 1, wherein the instructions when executed by the one or more processors, cause the wearable blood metrics measurement apparatus to perform the additional steps of:

identifying first candidate waves from the first signal data; identifying second candidate waves from the second signal data;
determining whether any of the first candidate waves satisfy a model match criterion to identify first high quality waves; and
determining whether any of the second candidate waves satisfy a model match criterion to identify second high quality waves;

and wherein the frequency transform function is applied to the first signal data of a first high quality wave and the second signal data of the second high-quality wave; wherein the first and phase value is for a first frequency component of the first signal data of the first high quality wave in the frequency domain; and wherein the second and phase value is for a first frequency component of the first signal data of the first high quality wave in the frequency domain.

3. The wearable blood metrics measurement apparatus (200) of claim 1, wherein the corresponding optical sensors of the wearable blood metrics measurement apparatus (200) are selected from one or more arrays of optical sensors (204).

4. The wearable blood metrics measurement apparatus (200) of claim 3, wherein the selecting at least two signal channels corresponding to the arterial signal includes:

obtaining time series data for each of a plurality of channel pairs associated with the one or more array of optical sensors (204);
calculating a correlation coefficient between each of the time series data;
determining a correlation value for each channel pair, each correlation value of a respective channel pair representing a correlation between a first and second channel of the respective channel pair;
selecting the channel pair having the highest correlation value relative to the correlation values of the other channel pairs of the plurality of channel pairs, the selected channel pair comprising the at least two signal channels corresponding to the at least two signal channels corresponding to the arterial signal.

5. The wearable blood metrics measurement apparatus (200) of claim 1, wherein the selecting at least two signal channels corresponding to the arterial signal further includes applying a high pass filter to remove selected motion artifacts prior to calculating the correlation coefficient.

6. The wearable blood metrics measurement apparatus (200) of claim 3, wherein the one or more arrays of optical sensors (204) comprise two rows of optical sensor pairs separated by a predetermined distance, and the pulse wave velocity is determined based on dividing the predetermined distance by the modified pulse transit time.

7. The wearable blood metrics measurement apparatus (200) of claim 1, wherein the obtaining the first signal data includes:

projecting, by an energy transmitter (304) of the wearable blood metrics measurement apparatus, energy at a first wavelength into tissue of a user;
generating the first signal data based on a first received portion of the energy at the first wavelength, the first received portion of energy being received through the tissue of the user.

8. The wearable blood metrics measurement apparatus (200) of claim 7, wherein the obtaining the second signal data includes:

projecting, by the energy transmitter (304) of the wearable blood metrics measurement apparatus, energy at a second wavelength into tissue of the user;

generating the second signal data based on a second received portion of the energy at the second wavelength, the second received portion of energy being received through the tissue of the user.

9. The wearable blood metrics measurement apparatus (200) of claim 8, wherein the first wavelength and the second wavelength comprise the same wavelength.

10. The wearable blood metrics measurement apparatus (200) of claim 1, wherein the first signal data and the second signal data each comprise photoplethysmogram (PPG) signal data.

11. A method (1000) comprising:

selecting (1002) at least two signal channels corresponding to an arterial signal, the at least two signal channels associated with corresponding optical sensors of a wearable blood metrics measurement apparatus (200) spatially separated by a predetermined distance;
obtaining (1004) first signal data from a first channel of the at least two signal channels over a predetermined period of time;
obtaining (1006) second signal data from a second channel of the at least two signal channels over the predetermined period of time;
determining a difference between the first signal data and a predetermined value;
determining a difference between the second signal data and the predetermined value;
if the difference between the first signal data and the predetermined value is greater than a threshold, ignoring that first signal data;
if the difference between the second signal data and the predetermined value is greater than the threshold, ignoring the second signal data;
applying (1008) a frequency transform function to each of the first signal data and the second signal data to transform the first signal data and the second signal data to a frequency domain;
determining (1010) a first phase value for a first frequency component of the first signal data in the frequency domain;
determining (1012) a second phase value for a second frequency component of the second signal data in the frequency domain;
determining (1014) a phase difference value between the first phase value and the second phase value;
determining (1016) a time shift value between the first signal data and the second signal data based on the phase difference value;
determining (1018) a modified pulse transit time based on the time shift value between the first signal data and the second signal data, the modified pulse transit time representing a transit time for a pressure wavefront to travel from a first optical sensor of the corresponding optical sensors to a second optical sensor of the corresponding optical sensors;
determining (1020) a pulse wave velocity based on the predetermined distance over the modified pulse transit time;
calculating (1022) an arterial blood pressure value based on the pulse wave velocity; and
providing (1024) a message including or being based on the arterial blood pressure value.

12. The method (1000) of claim 11, comprising the additional steps of: identifying first candidate waves from the first signal data; identifying second candidate waves from the second signal data;

determining whether any of the first candidate waves satisfy a model match criterion to identify first high quality waves; and
determining whether any of the second candidate waves satisfy a model match criterion to identify second high quality waves;
and wherein the frequency transform function is applied to the first signal data of a first high quality wave and the second signal data of the second high-quality wave; wherein the first and phase value is for a first frequency component of the first signal data of the first high quality wave in the frequency domain; and wherein the second and phase value is for a first frequency component of the first signal data of the first high quality wave in the frequency domain.

13. The method (1000) of claim 11, wherein the corresponding optical sensors of the wearable blood metrics measurement apparatus (200) are selected from one or more arrays of optical sensors (204).

**14.** The method (1000) of claim 12, wherein the selecting at least two signal channels corresponding to the arterial signal includes:

obtaining time series data for each of a plurality of channel pairs associated with the one or more array of optical sensors (204);
calculating a correlation coefficient between each of the time series data;
determining a correlation value for each channel pair, each correlation value of a respective channel pair representing a correlation between a first and second channel of the respective channel pair;
selecting the channel pair having the highest correlation value relative to the correlation values of the other channel pairs of the plurality of channel pairs, the selected channel pair comprising the at least two signal channels corresponding to the at least two signal channels corresponding to the arterial signal.

**15.** The method (1000) of claim 11, wherein the selecting at least two signal channels corresponding to the arterial signal further includes applying a high pass filter to remove selected motion artifacts prior to calculating the correlation coefficient,
or
wherein the obtaining the first signal data includes:

projecting, by an energy transmitter of the wearable blood metrics measurement apparatus, energy at a first wavelength into tissue of a user;
generating the first signal data based on a first received portion of the energy at the first wavelength, the first received portion of energy being received through the tissue of the user.

**16.** A non-transitory computer readable medium (1304) comprising instructions that, when executed, cause one or more processors (1302) to perform:

selecting at least two signal channels corresponding to an arterial signal, the at least two signal channels associated with corresponding optical sensors of the wearable blood metrics measurement apparatus (200) spatially separated by a predetermined distance;
obtaining first signal data from a first channel of the at least two signal channels over a predetermined period of time;
obtaining second signal data from a second channel of the at least two signal channels over the predetermined period of time;
determining a difference between the first signal data and a predetermined value;
determining a difference between the second signal data and the predetermined value;
if the difference between the first signal data and the predetermined value is greater than a threshold, ignoring that first signal data;
if the difference between the second signal data and the predetermined value is greater than the threshold, ignoring the second signal data;
applying a frequency transform function to each of the first signal data and the second signal data to transform the first signal data and the second signal data to a frequency domain;
determining a first phase value for a first frequency component of the first signal data in the frequency domain;
determining a second phase value for a second frequency component of the second signal data in the frequency domain;
determining a phase difference value between the first phase value and the second phase value; determining a time shift value between the first signal data and the second signal data based on the phase difference value;
determining a modified pulse transit time based on the time shift value between the first signal data and the second signal data, the modified pulse transit time representing a transit time for a pressure wavefront to travel from a first optical sensor of the corresponding optical sensors to a second optical sensor of the corresponding optical sensors;
determining a pulse wave velocity based on the predetermined distance over the modified pulse transit time;
calculating an arterial blood pressure value based on the pulse wave velocity; and
providing a message including or being based on the arterial blood pressure value.

**17.** The non-transitory computer readable medium (1304) of claim 16, wherein the instructions, when executed, further cause the processors (1302) to perform:

identifying first candidate waves from the first signal data;

identifying second candidate waves from the second signal data;

determining whether any of the first candidate waves satisfy a model match criterion to identify first high quality waves; and

determining whether any of the second candidate waves satisfy a model match criterion to identify second high quality waves;

and wherein the frequency transform function is applied to the first signal data of a first high quality wave and the second signal data of the second high-quality wave; wherein the first and phase value is for a first frequency component of the first signal data of the first high quality wave in the frequency domain; and wherein the second and phase value is for a first frequency component of the first signal data of the first high quality wave in the frequency domain.

18. The non-transitory computer readable medium (1304) of claim 16, further comprising instructions that, when executed, cause one or more processors (1302) to perform:

selecting the corresponding optical sensors of the wearable blood metrics measurement apparatus (200) from one or more arrays of optical sensors (204);

obtaining time series data for each of a plurality of channel pairs associated with the one or more array of optical sensors;

calculating a correlation coefficient between each of the time series data;

determining a correlation value for each channel pair, each correlation value of a respective channel pair representing a correlation between a first and second channel of the respective channel pair; and

selecting the channel pair having the highest correlation value relative to the correlation values of the other channel pairs of the plurality of channel pairs, the selected channel pair comprising the at least two signal channels corresponding to the at least two signal channels corresponding to the arterial signal.

**Patentansprüche**

1. Tragbares Blutmesswerte-Messgerät (200), umfassend:

einen oder mehrere Prozessoren (1302);

einen Speicher (1304), auf dem Anweisungen gespeichert sind, die bei Ausführung durch den einen oder die mehreren Prozessoren (1302) bewirken, dass das tragbare Blutmesswerte-Messgerät Folgendes ausführt:

Auswählen (1002) von zumindest zwei Signalkanälen, die entsprechenden optischen Sensoren des tragbaren Blutmesswerte-Messgeräts zugeordnet sind, die durch einen vorbestimmten Abstand räumlich getrennt sind;

Erhalten (1004) erster Signaldaten von einem ersten Kanal der zumindest zwei Signalkanäle über eine vorbestimmte Zeitdauer;

Erhalten (1006) zweiter Signaldaten von einem zweiten Kanal der zumindest zwei Signalkanäle über die vorbestimmte Zeitdauer;

Bestimmen eines Unterschieds zwischen den ersten Signaldaten und einem vorbestimmten Wert;

Bestimmen eines Unterschieds zwischen den zweiten Signaldaten und einem vorbestimmten Wert;

wenn der Unterschied zwischen den ersten Signaldaten und dem vorbestimmten Wert größer als eine Schwelle ist, Ignorieren der ersten Signaldaten;

wenn der Unterschied zwischen den zweiten Signaldaten und dem vorbestimmten Wert größer als die Schwelle ist, Ignorieren der zweiten Signaldaten;

Anwenden (1008) einer Frequenztransformationsfunktion auf alle aus den ersten Signaldaten und den zweiten Signaldaten, um die ersten Signaldaten und die zweiten Signaldaten in eine Frequenzdomäne umzuwandeln;

Bestimmen (1010) eines ersten Phasenwerts für eine erste Frequenzkomponente der ersten Signaldaten in der Frequenzdomäne;

Bestimmen (1012) eines zweiten Phasenwerts für eine zweite Frequenzkomponente der zweiten Signaldaten in der Frequenzdomäne;

Bestimmen (1014) eines Phasendifferenzwerts zwischen dem ersten Phasenwert und dem zweiten Phasenwert;

Bestimmen (1016) eines Zeitverschiebungswerts zwischen den ersten Signaldaten und den zweiten Signaldaten basierend auf dem Phasendifferenzwert;

Bestimmen einer modifizierten Pulswellenlaufzeit (1018) basierend auf dem Zeitverschiebungswert zwischen den ersten Signaldaten und den zweiten Signaldaten, wobei die modifizierte Pulswellenlaufzeit eine Laufzeit für eine Druckwellenfront ist, um von einem ersten optischen Sensor der entsprechenden optischen Sensoren zu einem zweiten optischen Sensor der entsprechenden optischen Sensoren zu wandern;
Bestimmen einer Pulswellengeschwindigkeit (1020) basierend auf der vorbestimmten Distanz über die modifizierte Pulswellenlaufzeit;
Berechnen eines arteriellen Blutdruckwerts (1022) basierend auf der Pulswellengeschwindigkeit; und
Bereitstellen (1024) einer Nachricht, die den arteriellen Blutdruckwert einschließt oder darauf basiert.

2. Tragbares Blutmesswerte-Messgerät (200) nach Anspruch 1, wobei die Anweisungen bei Ausführung durch den einen oder die mehreren Prozessoren bewirken, dass das tragbare Blutmesswerte-Messgerät die folgenden zusätzlichen Schritte ausführt:

Identifizieren erster Kandidatenwellen aus den ersten Signaldaten; Identifizieren zweiter Kandidatenwellen aus den zweiten Signaldaten;
Bestimmen, ob irgendwelche der ersten Kandidatenwellen ein Modellübereinstimmungskriterium erfüllen, um erste Wellen mit hoher Qualität zu identifizieren;
Bestimmen, ob irgendwelche der zweiten Kandidatenwellen ein Modellübereinstimmungskriterium erfüllen, um zweite Wellen mit hoher Qualität zu identifizieren;

und wobei die Frequenztransformationsfunktion auf die ersten Signaldaten einer ersten Welle mit hoher Qualität und auf die zweiten Signaldaten der zweiten Welle mit hoher Qualität angewandt wird; wobei der erste und Phasenwert für eine erste Frequenzkomponente der ersten Signaldaten der ersten Welle mit hoher Qualität in der Frequenzdomäne ist; und wobei der zweite und Phasenwert für eine erste Frequenzkomponente der ersten Signaldaten der ersten Welle mit hoher Qualität in der Frequenzdomäne ist.

3. Tragbares Blutmesswerte-Messgerät (200) nach Anspruch 1, wobei die entsprechenden optischen Sensoren des tragbaren Blutmesswerte-Messgeräts (200) aus einer oder mehreren Anordnungen von optischen Sensoren (204) ausgewählt sind.

4. Tragbares Blutmesswerte-Messgerät (200) nach Anspruch 3, wobei das Auswählen von zumindest zwei Signalkanälen, die dem arteriellen Signal entsprechen, umfasst:

Erhalten von Zeitreihendaten für jedes aus einer Vielzahl von Kanalpaaren, die der einen oder den mehreren Anordnungen von optischen Sensoren (204) zugeordnet sind;
Berechnen eines Korrelationskoeffizienten zwischen allen Zeitreihendaten;
Bestimmen eines Korrelationswerts für jedes Kanalpaar, wobei jeder Korrelationswert eines entsprechenden Kanalpaars eine Korrelation zwischen einem ersten und einem zweiten Kanal des entsprechenden Kanalpaars darstellt;
Auswählen des Kanalpaars mit dem höchsten Korrelationswert relativ zu den Korrelationswerten der anderen Kanalpaare der Vielzahl von Kanalpaaren, wobei das ausgewählte Kanalpaar zumindest die zwei Signalkanäle umfasst, die den zumindest zwei Signalkanälen entsprechen, die dem arteriellen Signal entsprechen.

5. Tragbares Blutmesswerte-Messgerät (200) nach Anspruch 1, wobei das Auswählen von zumindest zwei Signalkanälen, die dem arteriellen Signal entsprechen, außerdem das Anwenden eines Hochpassfilters umfassen, um ausgewählte Bewegungsartefakte vor der Berechnung des Korrelationskoeffizienten zu entfernen.

6. Tragbares Blutmesswerte-Messgerät (200) nach Anspruch 3, wobei die eine oder mehreren Anordnungen von optischen Sensoren (204) zwei Reihen von optischen Sensorpaaren umfassen, die durch einen vorbestimmten Abstand getrennt sind, und die Pulswellengeschwindigkeit basierend auf der Teilung des vorbestimmten Abstands durch die modifizierte Pulswellenlaufzeit bestimmt wird.

7. Tragbares Blutmesswerte-Messgerät (200) nach Anspruch 1, wobei das Erhalten der ersten Signaldaten umfasst:

Übertragen, mittels eines Energietransmitters (304) des tragbaren Blutmesswerte-Messgeräts, von Energie mit einer ersten Wellenlänge in das Gewebe eines Benutzers;
Erzeugen der ersten Signaldaten basierend auf einem ersten empfangenen Teil der Energie mit der ersten Wellenlänge, wobei der erste empfangene Teil der Energie durch das Gewebe des Benutzers empfangen wird.

8. Tragbares Blutmesswerte-Messgerät (200) nach Anspruch 7, wobei das Erhalten der zweiten Signaldaten umfasst:

Übertragen, mittels des Energietransmitters (304) des tragbaren Blutmesswerte-Messgeräts, von Energie mit einer zweiten Wellenlänge in das Gewebe eines Benutzers;
Erzeugen der zweiten Signaldaten basierend auf einem zweiten empfangenen Teil der Energie mit der zweiten Wellenlänge, wobei der zweite empfangene Teil der Energie durch das Gewebe des Benutzers empfangen wird.

9. Tragbares Blutmesswerte-Messgerät (200) nach Anspruch 8, wobei die erste Wellenlänge und die zweite Wellenlänge die gleiche Wellenlänge umfassen.

10. Tragbares Blutmesswerte-Messgerät (200) nach Anspruch 1, wobei die ersten Signaldaten und die zweiten Signaldaten jeweils Photoplethysmogramm(PPG)-Signaldaten umfassen.

11. Verfahren (1000), umfassend:

Auswählen (1002) von zumindest zwei Signalkanälen, die einem arteriellen Signal entsprechen, wobei die zumindest zwei Signalkanäle entsprechenden optischen Sensoren eines tragbaren Blutmessdaten-Messgeräts (200) zugeordnet sind, die durch einen vorbestimmten Abstand räumlich getrennt sind;
Erhalten (1004) erster Signaldaten von einem ersten Kanal der zumindest zwei Signalkanäle über eine vorbestimmte Zeitdauer;
Erhalten (1006) zweiter Signaldaten von einem zweiten Kanal der zumindest zwei Signalkanäle über die vorbestimmte Zeitdauer;
Bestimmen eines Unterschieds zwischen den ersten Signaldaten und einem vorbestimmten Wert;
Bestimmen eines Unterschieds zwischen den zweiten Signaldaten und dem vorbestimmten Wert;
wenn der Unterschied zwischen den ersten Signaldaten und dem vorbestimmten Wert größer als eine Schwelle ist, Ignorieren der ersten Signaldaten;
wenn der Unterschied zwischen den zweiten Signaldaten und dem vorbestimmten Wert größer als die Schwelle ist, Ignorieren der zweiten Signaldaten;
Anwenden (1008) einer Frequenztransformationsfunktion auf alle aus den ersten Signaldaten und den zweiten Signaldaten, um die ersten Signaldaten und die zweiten Signaldaten in eine Frequenzdomäne umzuwandeln;
Bestimmen (1010) eines ersten Phasenwerts für eine erste Frequenzkomponente der ersten Signaldaten in der Frequenzdomäne;
Bestimmen (1012) eines zweiten Phasenwerts für eine zweite Frequenzkomponente der zweiten Signaldaten in der Frequenzdomäne;
Bestimmen (1014) eines Phasendifferenzwerts zwischen dem ersten Phasenwert und dem zweiten Phasenwert;
Bestimmen (1016) eines Zeitverschiebungswerts zwischen den ersten Signaldaten und den zweiten Signaldaten basierend auf dem Phasendifferenzwert;
Bestimmen (1018) einer modifizierten Pulswellenlaufzeit basierend auf dem Zeitverschiebungswert zwischen den ersten Signaldaten und den zweiten Signaldaten, wobei die modifizierte Pulswellenlaufzeit eine Laufzeit für eine Druckwellenfront darstellt, um von einem ersten optischen Sensor der entsprechenden optischen Sensoren zu einem zweiten optischen Sensor der entsprechenden optischen Sensoren zu wandern;
Bestimmen (1020) einer Pulswellengeschwindigkeit basierend auf dem vorbestimmten Abstand über die modifizierte Pulswellenlaufzeit;
Berechnen (1022) eines arteriellen Blutdruckwerts basierend auf der Pulswellengeschwindigkeit; und
Bereitstellen (1024) einer Nachricht, die den arteriellen Blutdruckwert einschließt oder darauf basiert.

12. Verfahren (1000) nach Anspruch 11, das die folgenden zusätzlichen Schritte umfasst: Identifizieren erster Kandidatenwellen aus den ersten Signaldaten; Identifizieren zweiter Kandidatenwellen aus den zweiten Signaldaten;

Bestimmen, ob irgendwelche der ersten Kandidatenwellen ein Modellübereinstimmungskriterium erfüllen, um erste Wellen mit hoher Qualität zu identifizieren;
Bestimmen ob irgendwelche der zweiten Kandidatenwellen ein Modellübereinstimmungskriterium erfüllen, um zweite Wellen mit hoher Qualität zu identifizieren;
und wobei die Frequenztransformationsfunktion auf die ersten Signaldaten einer ersten Welle mit hoher Qualität und die zweiten Signaldaten der zweiten Welle mit hoher Qualität angewandt wird; wobei der erste und Phasenwert für eine erste Frequenzkomponente der ersten Signaldaten der ersten Welle mit hoher Qualität in der Frequenzdomäne ist; und wobei der zweite und Phasenwert für eine erste Frequenzkomponente der ersten Signaldaten der ersten Welle mit hoher Qualität in der Frequenzdomäne ist.

**13.** Verfahren (1000) nach Anspruch 11, wobei die entsprechenden optischen Sensoren des tragbaren Blutmesswerte-Messgeräts (200) aus einer oder mehreren Anordnungen von optischen Sensoren (204) ausgewählt sind.

**14.** Verfahren (1000) nach Anspruch 12, wobei das Auswählen von zumindest zwei Signalkanälen, die dem arteriellen Signal entsprechen, umfasst:

Erhalten von Zeitreihendaten für jedes aus einer Vielzahl von Kanalpaaren, die der einen oder den mehreren Anordnungen von optischen Sensoren (204) zugeordnet sind;
Berechnen eines Korrelationskoeffizienten zwischen allen Zeitreihendaten;
Bestimmen eines Korrelationswerts für jedes Kanalpaar, wobei jeder Korrelationswert eines entsprechenden Kanalpaars eine Korrelation zwischen einem ersten und einem zweiten Kanal des entsprechenden Kanalpaars darstellt;
Auswählen des Kanalpaars mit dem höchsten Korrelationswert relativ zu den Korrelationswerten der anderen Kanalpaare der Vielzahl von Kanalpaaren, wobei das ausgewählte Kanalpaar zumindest die zwei Signalkanäle umfasst, die den zumindest zwei Signalkanälen entsprechen, die dem arteriellen Signal entsprechen.

**15.** Verfahren (1000) nach Anspruch 11, wobei das Auswählen von zumindest zwei Signalkanälen, die dem arteriellen Signal entsprechen, außerdem das Anwenden eines Hochpassfilters umfassen, um ausgewählte Bewegungsartefakte vor der Berechnung des Korrelationskoeffizienten zu entfernen,
oder
wobei das Erhalten der ersten Signaldaten umfasst:

Übertragen, mittels eines Energietransmitters des tragbaren Blutmesswerte-Messgeräts, von Energie mit einer ersten Wellenlänge in das Gewebe eines Benutzers;
Erzeugen der ersten Signaldaten basierend auf einem ersten empfangenen Teil der Energie mit der ersten Wellenlänge, wobei der erste empfangene Teil der Energie durch das Gewebe des Benutzers empfangen wird.

**16.** Nichttransitorisches, computerlesbares Medium (1304), das Anweisungen umfasst, die bei ihrer Ausführung bewirken, dass ein oder mehrere Prozessoren (1302) Folgendes ausführen:

Auswählen von zumindest zwei Signalkanälen, die einem arteriellen Signal entsprechen, wobei die zumindest zwei Signalkanäle entsprechenden optischen Sensoren eines tragbaren Blutmessdaten-Messgeräts (200) zugeordnet sind, die durch einen vorbestimmten Abstand räumlich getrennt sind;
Erhalten erster Signaldaten von einem ersten Kanal der zumindest zwei Signalkanäle über eine vorbestimmte Zeitdauer;
Erhalten zweiter Signaldaten von einem zweiten Kanal der zumindest zwei Signalkanäle über die vorbestimmte Zeitdauer;
Bestimmen eines Unterschieds zwischen den ersten Signaldaten und einem vorbestimmten Wert;
Bestimmen eines Unterschieds zwischen den zweiten Signaldaten und dem vorbestimmten Wert;
wenn der Unterschied zwischen den ersten Signaldaten und dem vorbestimmten Wert größer als eine Schwelle ist, Ignorieren der ersten Signaldaten;
wenn der Unterschied zwischen den zweiten Signaldaten und dem vorbestimmten Wert größer als die Schwelle ist, Ignorieren der zweiten Signaldaten;
Anwenden einer Frequenztransformationsfunktion auf alle aus den ersten Signaldaten und den zweiten Signaldaten, um die ersten Signaldaten und die zweiten Signaldaten in eine Frequenzdomäne umzuwandeln;
Bestimmen eines ersten Phasenwerts für eine erste Frequenzkomponente der ersten Signaldaten in der Frequenzdomäne;
Bestimmen eines zweiten Phasenwerts für eine zweite Frequenzkomponente der zweiten Signaldaten in der Frequenzdomäne;
Bestimmen eines Phasendifferenzwerts zwischen dem ersten Phasenwert und dem zweiten Phasenwert; Bestimmen eines Zeitverschiebungswerts zwischen den ersten Signaldaten und den zweiten Signaldaten basierend auf dem Phasendifferenzwert;
Bestimmen einer modifizierten Pulswellenlaufzeit basierend auf dem Zeitverschiebungswert zwischen den ersten Signaldaten und den zweiten Signaldaten, wobei die modifizierte Pulswellenlaufzeit eine Laufzeit für eine Druckwellenfront darstellt, um von einem ersten optischen Sensor der entsprechenden optischen Sensoren zu einem zweiten optischen Sensor der entsprechenden optischen Sensoren zu wandern;
Bestimmen einer Pulswellengeschwindigkeit basierend auf dem vorbestimmten Abstand über die modifizierte Pulswellenlaufzeit;

Berechnen eines arteriellen Blutdruckwerts basierend auf der Pulswellengeschwindigkeit; und

Bereitstellen einer Nachricht, die den arteriellen Blutdruckwert einschließt oder darauf basiert.

**17.** Nichttransitorisches, computerlesbares Medium (1304) nach Anspruch 16, wobei die Anweisungen bei ihrer Ausführung weiters bewirken, dass die Prozessoren (1302) Folgendes ausführen:

Identifizieren erster Kandidatenwellen aus den ersten Signaldaten;

Identifizieren zweiter Kandidatenwellen aus den zweiten Signaldaten;

Bestimmen, ob irgendwelche der ersten Kandidatenwellen ein Modellübereinstimmungskriterium erfüllen, um erste Wellen mit hoher Qualität zu identifizieren;

Bestimmen, ob irgendwelche der zweiten Kandidatenwellen ein Modellübereinstimmungskriterium erfüllen, um zweite Wellen mit hoher Qualität zu identifizieren;

und wobei die Frequenztransformationsfunktion auf die ersten Signaldaten einer ersten Welle mit hoher Qualität und auf die zweiten Signaldaten der zweiten Welle mit hoher Qualität angewandt wird; wobei der erste und Phasenwert für eine erste Frequenzkomponente der ersten Signaldaten der ersten Welle mit hoher Qualität in der Frequenzdomäne ist; und wobei der zweite und Phasenwert für eine erste Frequenzkomponente der ersten Signaldaten der ersten Welle mit hoher Qualität in der Frequenzdomäne ist.

**18.** Nichttransitorisches, computerlesbares Medium (1304) nach Anspruch 16, das außerdem Anweisungen umfasst, die bei ihrer Ausführung bewirken, dass der eine die oder mehreren Prozessoren (1302) Folgendes ausführen:

Auswählen der entsprechenden optischen Sensoren des tragbaren Blutmesswerte-Messgeräts (200) aus einer oder mehreren Anordnungen von optischen Sensoren (204);

Erhalten von Zeitreihendaten für jedes aus einer Vielzahl von Kanalpaaren, die der einen oder den mehreren Anordnungen von optischen Sensoren zugeordnet sind;

Berechnen eines Korrelationskoeffizienten zwischen allen Zeitreihendaten;

Bestimmen eines Korrelationswerts für jedes Kanalpaar, wobei jeder Korrelationswert eines entsprechenden Kanalpaars eine Korrelation zwischen einem ersten und einem zweiten Kanal des entsprechenden Kanalpaars darstellt;

Auswählen des Kanalpaars mit dem höchsten Korrelationswert relativ zu den Korrelationswerten der anderen Kanalpaare der Vielzahl von Kanalpaaren, wobei das ausgewählte Kanalpaar zumindest die zwei Signalkanäle umfasst, die den zumindest zwei Signalkanälen entsprechen, die dem arteriellen Signal entsprechen.

## Revendications

**1.** Appareil de mesure de paramètres sanguins portable (200) comprenant :

un ou plusieurs processeurs (1302) ;

une mémoire (1304) stockant des instructions qui, lorsqu'elles sont exécutées par les un ou plusieurs processeurs (1302), amènent l'appareil de mesure de paramètres sanguins portable à exécuter :

la sélection (1002) d'au moins deux canaux de signal correspondant à un signal artériel, les au moins deux canaux de signal étant associés à des capteurs optiques correspondants de l'appareil de mesure de paramètres sanguins portable séparés spatialement par une distance prédéterminée ;

l'obtention (1004) de premières données de signal à partir d'un premier canal des au moins deux canaux de signal pendant une période prédéterminée ;

l'obtention (1006) de secondes données de signal à partir d'un second canal des au moins deux canaux de signal pendant la période prédéterminée ;

la détermination d'une différence entre les premières données de signal et une valeur prédéterminée ;

la détermination d'une différence entre les secondes données de signal et la valeur prédéterminée ;

si la différence entre les premières données de signal et la valeur prédéterminée est supérieure à un seuil, le fait d'ignorer ces premières données de signal ;

si la différence entre les secondes données de signal et la valeur prédéterminée est supérieure au seuil, le fait d'ignorer les secondes données de signal ;

l'application (1008) d'une fonction de transformation de fréquence à chacune des premières données de signal et des secondes données de signal pour transformer les premières données de signal et les secondes données de signal en un domaine fréquentiel ;

la détermination (1010) d'une première valeur de phase pour une première composante de fréquence des premières données de signal dans le domaine fréquentiel ;

la détermination (1012) d'une seconde valeur de phase pour une seconde composante de fréquence des secondes données de signal dans le domaine fréquentiel ;

la détermination (1014) d'une valeur de différence de phase entre la première valeur de phase et la seconde valeur de phase ;

la détermination (1016) d'une valeur de décalage temporel entre les premières données de signal et les secondes données de signal sur la base de la valeur de différence de phase ;

la détermination d'une durée de transit d'impulsion modifiée (1018) sur la base de la valeur de décalage temporel entre les premières données de signal et les secondes données de signal, la durée de transit d'impulsion modifiée représentant une durée de transit pour qu'un front d'onde de pression se déplace d'un premier capteur optique des capteurs optiques correspondants à un second capteur optique des capteurs optiques correspondants ;

la détermination d'une vitesse d'onde d'impulsion (1020) sur la base de la distance prédéterminée pendant la durée de transit d'impulsion modifiée ;

le calcul d'une valeur de pression sanguine artérielle (1022) sur la base de la vitesse d'onde d'impulsion ;

et la fourniture (1024) d'un message comportant ou étant basé sur la valeur de pression sanguine artérielle.

2.  Appareil de mesure de paramètres sanguins portable (200) selon la revendication 1, dans lequel les instructions, lorsqu'elles sont exécutées par les un ou plusieurs processeurs, amènent l'appareil de mesure de paramètres sanguins portable à exécuter les étapes supplémentaires suivantes :

    l'identification de premières ondes candidates à partir des premières données de signal ; l'identification de secondes ondes candidates à partir des secondes données de signal ;

    le fait de déterminer si l'une quelconque des premières ondes candidates satisfait à un critère de correspondance de modèle pour identifier des premières ondes de haute qualité ; et

    le fait de déterminer si l'une quelconque des secondes ondes candidates satisfait à un critère de correspondance de modèle pour identifier des secondes ondes de haute qualité ; et

    dans lequel la fonction de transformation de fréquence est appliquée aux premières données de signal d'une première onde de haute qualité et aux secondes données de signal de la seconde onde de haute qualité ; dans lequel la première valeur de phase est destinée à une première composante de fréquence des premières données de signal de la première onde de haute qualité dans le domaine fréquentiel ; et dans lequel la seconde valeur de phase est destinée à une première composante de fréquence des premières données de signal de la première onde de haute qualité dans le domaine fréquentiel.

3.  Appareil de mesure de paramètres sanguins portable (200) selon la revendication 1, dans lequel les capteurs optiques correspondants de l'appareil de mesure de paramètres sanguins portable (200) sont sélectionnés à partir d'un ou de plusieurs réseaux de capteurs optiques (204) .

4.  Appareil de mesure de paramètres sanguins portable (200) selon la revendication 3, dans lequel la sélection d'au moins deux canaux de signal correspondant au signal artériel comporte :

    l'obtention de données de séries chronologiques pour chacune d'une pluralité de paires de canaux associées aux un ou plusieurs réseaux de capteurs optiques (204) ;

    le calcul d'un coefficient de corrélation entre chacune des données de séries chronologiques ;

    la détermination d'une valeur de corrélation pour chaque paire de canaux, chaque valeur de corrélation d'une paire de canaux respective représentant une corrélation entre un premier et un second canal de la paire de canaux respective ;

    la sélection de la paire de canaux ayant la valeur de corrélation la plus élevée par rapport aux valeurs de corrélation des autres paires de canaux de la pluralité de paires de canaux, la paire de canaux sélectionnée comprenant les au moins deux canaux de signal correspondant aux au moins deux canaux de signal correspondant au signal artériel.

5.  Appareil de mesure de paramètres sanguins portable (200) selon la revendication 1, dans lequel la sélection d'au moins deux canaux de signal correspondant au signal artériel comporte en outre l'application d'un filtre passe-haut pour supprimer des artefacts de mouvement sélectionnés avant de calculer le coefficient de corrélation.

6.  Appareil de mesure de paramètres sanguins portable (200) selon la revendication 3, dans lequel les un ou plusieurs réseaux de capteurs optiques (204) comprennent deux rangées de paires de capteurs optiques séparées par une distance prédéterminée, et la vitesse d'onde d'impulsion est déterminée sur la base de la division de la distance prédéterminée par la durée de transit d'impulsion modifiée.

7. Appareil de mesure de paramètres sanguins portable (200) selon la revendication 1, dans lequel l'obtention des premières données de signal comporte :

la projection, par un émetteur d'énergie (304) de l'appareil de mesure de paramètres sanguins portable, d'énergie à une première longueur d'onde dans le tissu d'un utilisateur ;
la génération des premières données de signal sur la base d'une première partie reçue de l'énergie à la première longueur d'onde, la première partie reçue de l'énergie étant reçue à travers le tissu de l'utilisateur.

8. Appareil de mesure de paramètres sanguins portable (200) selon la revendication 7, dans lequel l'obtention des secondes données de signal comporte :

la projection, par l'émetteur d'énergie (304) de l'appareil de mesure des paramètres sanguins portable, d'énergie à une seconde longueur d'onde dans le tissu de l'utilisateur ;
la génération des secondes données de signal sur la base d'une seconde partie reçue de l'énergie à la seconde longueur d'onde, la seconde partie reçue de l'énergie étant reçue à travers le tissu de l'utilisateur.

9. Appareil de mesure de paramètres sanguins portable (200) selon la revendication 8, dans lequel la première longueur d'onde et la seconde longueur d'onde comprennent la même longueur d'onde.

10. Appareil de mesure de paramètres sanguins portable (200) selon la revendication 1, dans lequel les premières données de signal et les secondes données de signal comprennent chacune des données de signal de photopléthysmogramme (PPG).

11. Procédé (1000) comprenant :

la sélection (1002) d'au moins deux canaux de signal correspondant à un signal artériel, les au moins deux canaux de signal étant associés à des capteurs optiques correspondants d'un appareil de mesure de paramètres sanguins portable (200) séparés spatialement par une distance prédéterminée ;
l'obtention (1004) de premières données de signal à partir d'un premier canal des au moins deux canaux de signal pendant une période prédéterminée ;
l'obtention (1006) de secondes données de signal à partir d'un second canal des au moins deux canaux de signal pendant la période prédéterminée ;
la détermination d'une différence entre les premières données de signal et une valeur prédéterminée ;
la détermination d'une différence entre les secondes données de signal et la valeur prédéterminée ;
si la différence entre les premières données de signal et la valeur prédéterminée est supérieure à un seuil, le fait d'ignorer ces premières données de signal ;
si la différence entre les secondes données de signal et la valeur prédéterminée est supérieure au seuil, le fait d'ignorer les secondes données de signal ;
l'application (1008) d'une fonction de transformation de fréquence à chacune des premières données de signal et des secondes données de signal pour transformer les premières données de signal et les secondes données de signal en un domaine fréquentiel ;
la détermination (1010) d'une première valeur de phase pour une première composante de fréquence des premières données de signal dans le domaine fréquentiel ;
la détermination (1012) d'une seconde valeur de phase pour une seconde composante de fréquence des secondes données de signal dans le domaine fréquentiel;
la détermination (1014) d'une valeur de différence de phase entre la première valeur de phase et la seconde valeur de phase ;
la détermination (1016) d'une valeur de décalage temporel entre les premières données de signal et les secondes données de signal sur la base de la valeur de différence de phase ;
la détermination (1018) d'une durée de transit d'impulsion modifiée sur la base de la valeur de décalage temporel entre les premières données de signal et les secondes données de signal, la durée de transit d'impulsion modifiée représentant une durée de transit pour qu'un front d'onde de pression se déplace d'un premier capteur optique des capteurs optiques correspondants à un second capteur optique des capteurs optiques correspondants ;

la détermination (1020) d'une vitesse d'onde d'impulsion sur la base de la distance prédéterminée pendant la durée de transit d'impulsion modifiée ;

le calcul (1022) d'une valeur de pression sanguine artérielle sur la base de la vitesse d'onde d'impulsion ; et

la fourniture (1024) d'un message comportant ou étant basé sur la valeur de pression sanguine artérielle.

12. Procédé (1000) selon la revendication 11, comprenant les étapes supplémentaires suivantes :

l'identification de premières ondes candidates à partir des premières données de signal ;

l'identification de secondes ondes candidates à partir des secondes données de signal ;

le fait de déterminer si l'une quelconque des premières ondes candidates satisfait à un critère de correspondance de modèle pour identifier des premières ondes de haute qualité ; et

le fait de déterminer si l'une quelconque des secondes ondes candidates satisfait à un critère de correspondance de modèle pour identifier des secondes ondes de haute qualité ; et dans lequel la fonction de transformation de fréquence est appliquée aux premières données de signal d'une première onde de haute qualité et aux secondes données de signal de la seconde onde de haute qualité ; dans lequel la première valeur de phase est destinée à une première composante de fréquence des premières données de signal de la première onde de haute qualité dans le domaine fréquentiel ; et dans lequel la seconde valeur de phase est destinée à une première composante de fréquence des premières données de signal de la première onde de haute qualité dans le domaine fréquentiel.

13. Procédé (1000) selon la revendication 11, dans lequel les capteurs optiques correspondants de l'appareil de mesure de paramètres sanguins portable (200) sont sélectionnés à partir d'un ou de plusieurs réseaux de capteurs optiques (204).

14. Procédé (1000) selon la revendication 12, dans lequel la sélection d'au moins deux canaux de signal correspondant au signal artériel comporte :

l'obtention de données de séries chronologiques pour chacune d'une pluralité de paires de canaux associées aux un ou plusieurs réseaux de capteurs optiques (204) ;

le calcul d'un coefficient de corrélation entre chacune des données de séries chronologiques ;

la détermination d'une valeur de corrélation pour chaque paire de canaux, chaque valeur de corrélation d'une paire de canaux respective représentant une corrélation entre un premier et un second canal de la paire de canaux respective ;

la sélection de la paire de canaux ayant la valeur de corrélation la plus élevée par rapport aux valeurs de corrélation des autres paires de canaux de la pluralité de paires de canaux, la paire de canaux sélectionnée comprenant les au moins deux canaux de signal correspondant aux au moins deux canaux de signal correspondant au signal artériel.

15. Procédé (1000) selon la revendication 11, dans lequel la sélection d'au moins deux canaux de signal correspondant au signal artériel comporte en outre l'application d'un filtre passe-haut pour supprimer des artefacts de mouvement sélectionnés avant de calculer le coefficient de corrélation, ou

dans lequel l'obtention des premières données de signal comporte :

la projection, par un émetteur d'énergie de l'appareil de mesure de paramètres sanguins portable, d'énergie à une première longueur d'onde dans le tissu d'un utilisateur ;

la génération des premières données de signal sur la base d'une première partie reçue de l'énergie à la première longueur d'onde, la première partie reçue de l'énergie étant reçue à travers le tissu de l'utilisateur.

16. Support non transitoire lisible par ordinateur (1304) comprenant des instructions qui, lorsqu'elles sont exécutées, amènent un ou plusieurs processeurs (1302) à exécuter :

la sélection d'au moins deux canaux de signal correspondant à un signal artériel, les au moins deux canaux de signal étant associés à des capteurs optiques correspondants de l'appareil de mesure de paramètres sanguins portable (200) séparés spatialement par une distance prédéterminée ;

l'obtention de premières données de signal à partir d'un premier canal des au moins deux canaux de signal pendant une période prédéterminée ;

l'obtention de secondes données de signal à partir d'un second canal des au moins deux canaux de signal pendant la période prédéterminée ;

la détermination d'une différence entre les premières données de signal et une valeur prédéterminée ;

la détermination d'une différence entre les secondes données de signal et la valeur prédéterminée ;

si la différence entre les premières données de signal et la valeur prédéterminée est supérieure à un seuil, le fait d'ignorer ces premières données de signal ;

si la différence entre les secondes données de signal et la valeur prédéterminée est supérieure au seuil, le fait d'ignorer les secondes données de signal ;

l'application d'une fonction de transformation de fréquence à chacune des premières données de signal et des secondes données de signal pour transformer les premières données de signal et les secondes données de signal en un domaine fréquentiel ;

la détermination d'une première valeur de phase pour une première composante de fréquence des premières données de signal dans le domaine fréquentiel ;

la détermination d'une seconde valeur de phase pour une seconde composante de fréquence des secondes données de signal dans le domaine fréquentiel ;

la détermination d'une valeur de différence de phase entre la première valeur de phase et la seconde valeur de phase ; la détermination d'une valeur de décalage temporel entre les premières données de signal et les secondes données de signal sur la base de la valeur de différence de phase ;

la détermination d'une durée de transit d'impulsion modifiée sur la base de la valeur de décalage temporel entre les premières données de signal et les secondes données de signal, la durée de transit d'impulsion modifiée représentant une durée de transit pour qu'un front d'onde de pression se déplace d'un premier capteur optique des capteurs optiques correspondants à un second capteur optique des capteurs optiques correspondants ;

la détermination d'une vitesse d'onde d'impulsion sur la base de la distance prédéterminée pendant la durée de transit d'impulsion modifiée ;

le calcul d'une valeur de pression sanguine artérielle sur la base de la vitesse d'onde d'impulsion ; et

la fourniture d'un message comportant ou étant basé sur la valeur de pression sanguine artérielle.

17. Support non transitoire lisible par ordinateur (1304) selon la revendication 16, dans lequel les instructions, lorsqu'elles sont exécutées, amènent en outre les processeurs (1302) à exécuter :

l'identification de premières ondes candidates à partir des premières données de signal ;

l'identification de secondes ondes candidates à partir des secondes données de signal ;

le fait de déterminer si l'une quelconque des premières ondes candidates satisfait à un critère de correspondance de modèle pour identifier des premières ondes de haute qualité ; et

le fait de déterminer si l'une quelconque des secondes ondes candidates satisfait à un critère de correspondance de modèle pour identifier des secondes ondes de haute qualité ; et

dans lequel la fonction de transformation de fréquence est appliquée aux premières données de signal d'une première onde de haute qualité et aux secondes données de signal de la seconde onde de haute qualité ; dans lequel la première valeur de phase est destinée à une première composante de fréquence des premières données de signal de la première onde de haute qualité dans le domaine fréquentiel ; et dans lequel la seconde valeur de phase est destinée à une première composante de fréquence des premières données de signal de la première onde de haute qualité dans le domaine fréquentiel.

18. Support non transitoire lisible par ordinateur (1304) selon la revendication 16, comprenant en outre des instructions qui, lorsqu'elles sont exécutées, amènent un ou plusieurs processeurs (1302) à exécuter :

la sélection des capteurs optiques correspondants de l'appareil de mesure de paramètres sanguins portable (200) à partir d'un ou de plusieurs réseaux de capteurs optiques (204) ;

l'obtention de données de séries chronologiques pour chacune d'une pluralité de paires de canaux associées aux un ou plusieurs réseaux de capteurs optiques ;

le calcul d'un coefficient de corrélation entre chacune des données de séries chronologiques ;

la détermination d'une valeur de corrélation pour chaque paire de canaux, chaque valeur de corrélation d'une paire de canaux respective représentant une corrélation entre un premier et un second canal de la paire de canaux respective ; et

la sélection de la paire de canaux ayant la valeur de corrélation la plus élevée par rapport aux valeurs de corrélation des autres paires de canaux de la pluralité de paires de canaux, la paire de canaux sélectionnée comprenant les au moins deux canaux de signal correspondant aux au moins deux canaux de signal correspondant au signal artériel.

100

ECG ▲▼

PTT

104 102

Plethysmo ▲▼

106

FIG. 2

FIG. 3A

Blood Metrics Measurement Apparatus 902

Communication Module 320

Analyzer 302

Motion Sensor 312

Blood Pressure Calculation System 314

Registration Module 318

Energy Transmitter 304

ENERGY RECEIVER 306

Pressure Sensor 308

User Interface Module 316

300

310

351

352

**360**

364

366

top row        bottom row

362

sensor array

radial artery

wrist

Δx

368    PWV = Δx/PTT

FIG. 3B

**FIG. 3C**

(a)

ppg signals from sensor array

ppg

top row                    bottom row

(b)

**400**

FIG. 4

500

Project energy into tissue of user
502

Receive portion of energy through the tissue of user
504

Generate one or more signals based on received portion of energy
506

Analyze one or more signals
508

Provide one or more signals for blood pressure calculation
510

FIG. 5

600

```
┌─────────────────────────────────────────────────────────┐
│          Register blood metrics measurement apparatus     │
│                            602                            │
└─────────────────────────────────────────────────────────┘
                              │
                              ▼
┌─────────────────────────────────────────────────────────┐
│                  Receive one or more signals              │
│                            604                            │
└─────────────────────────────────────────────────────────┘
                              │
                              ▼
┌─────────────────────────────────────────────────────────┐
│      Calculate blood pressure value based on one or more signals │
│                            606                            │
└─────────────────────────────────────────────────────────┘
                              │
                              ▼
┌─────────────────────────────────────────────────────────┐
│              Present blood pressure message to a user     │
│                            608                            │
└─────────────────────────────────────────────────────────┘
```

FIG. 6

Blood Pressure Calculation System 314

| Management Module 702 | Signal Database 704<br><br>Signal Records 728 | Wave Database 706<br><br>Wave Records 730 | Wave Feature Database 708<br><br>Wave Feature Records 732 | Feature Vector Database 710<br><br>Feature Vector Records 734 |

| BP Model Database 712<br><br>BP Model Records 736 | BP Results Database 714<br><br>BP Results Records 738 | Rules Database 716 | Pre-Processing Module 718 |

Rules Database 716

| Pre-Processing Rules 740 | Message Rules 748 |
| Wave Selection Rules 742 | Channel Selection Rules 750 |
| Feature Extraction Rules 744 | PTTM Rules 752 |
| BP Processing Rules 746 | |

| Wave Selection Module 720 | Feature Extraction Module 722 |

| Blood Pressure Processing Module 724 | Communication Module 726 |

EP 3 419 511 B1

FIG. 7

800

| Obtains data over time from channels to create data segments | 802 |

↓

| Filter data segments to remove large motion artifacts | 804 |

↓

| Determine correlation between data segments | 806 |

↓

| Select channel pair that provides highest correlation | 808 |

FIG. 8

FIG. 9

1000

Selecting at least two signal channels corresponding to an arterial signal, the at least two signal channels associated with corresponding optical sensors of the wearable blood pressure calculation apparatus 1002

Obtaining first signal data from a first channel of the at least two signal channels over a predetermined period of time 1004

Obtaining second signal data from a second channel of the at least two signal channels over the predetermined period of time 1006

Applying a frequency transform function to each of the first signal data and the second signal data to transform the first signal data and the second signal data to a frequency domain 1008

Determining a first phase value for a first frequency component of the first signal data in the frequency domain 1010

Determining a second phase value for a second frequency component of the second signal data in the frequency domain 1012

Determining a phase difference value between the first phase value and the second phase value 1014

Determining a time shift value between the first signal data and the second signal data based on the phase difference value 1016

Determining a modified pulse transmit time based on the time shift value between the first signal data and the second signal data 1018

Determining a pulse wave velocity based on the modified pulse transit time 1020

Calculating an arterial blood pressure value based on the pulse wave velocity 1022

Providing a message including or being based on the arterial blood pressure value 1024

FIG. 10

1100

| Apply Fourier transform to each of selected data segments to create transformed data segments | ⟋ 1102 |

| Calculate sum of squared amplitude to find estimate of heart rate as frequency that corresponds to largest amplitude of combined spectrum | ⟋ 1104 |

| Apply inverse tangent function on imaginary and real parts of transformed data segments to create phase value for each frequency component | ⟋ 1106 |

| Determine phase differences for frequency components at or near heart rate | ⟋ 1108 |

| Filter and combine computed phase differences | ⟋ 1110 |

FIG. 11

1200

Delay one of two signals by a specific number of samples — 1202

Create window of points over a time period for two signals — 1204

Compute cost function at each relative shift of two signals to find modified pulse transit time — 1206

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080294058 A1
- US 20130137938 A1